(19) Europäisches Patentamt
European Patent Office
Office européen des brevets

(11) EP 1 466 969 A1

(12) EUROPEAN PATENT APPLICATION
published in accordance with Art. 158(3) EPC

(43) Date of publication:
**13.10.2004 Bulletin 2004/42**

(21) Application number: **03701755.5**

(22) Date of filing: **17.01.2003**

(51) Int Cl.[7]: **C12N 9/42**, C07H 11/00, C08B 37/00, C12P 19/04

(86) International application number:
**PCT/JP2003/000334**

(87) International publication number:
**WO 2003/062412 (31.07.2003 Gazette 2003/31)**

(84) Designated Contracting States:
**AT BE BG CH CY CZ DE DK EE ES FI FR GB GR IE IT LI LU MC NL PT SE SI SK TR**
Designated Extension States:
**AL LT LV MK RO**

(30) Priority: **18.01.2002 JP 2002010844**
**23.05.2002 JP 2002149874**

(71) Applicant: **TAKARA BIO INC.**
**Otsu-shi, Shiga 520-2193 (JP)**

(72) Inventors:
- **SAKAI, Takeshi**
  **Kusatsu-shi, Shiga 525-0072 (JP)**
- **AMARUME, Hitomi**
  **Hirosaki-shi, Aomori 036-8255 (JP)**
- **IKAI, Katsushige**
  **Koka-gun, Shiga 520-3332 (JP)**
- **KATO, Ikunoshin**
  **Koka-gun, Shiga 529-1851 (JP)**

(74) Representative: **Schnappauf, Georg Dr. et al**
**Dr. Volker Vossius**
**Patent- und Rechtsanwaltskanzlei**
**Geibelstrasse 6**
**81679 München (DE)**

(54) **SULFATED FUCAN**

(57) An enzyme capable of degrading sulfated fucan originating in a brown alga of Fucales which is useful in sugar chain engineering; a process for producing this enzyme; an oligosaccharide obtained by treating sulfated fucan with the enzyme; and production thereof.

EP 1 466 969 A1

## Description

Technical Field

The present invention relates to an enzyme that degrades a sulfated fucan which is useful in a field of glycotechnology and a method for producing the enzyme, as well as a sulfated fucan oligosaccharide which is useful as a reagent for glycotechnology and a method for producing the same.

Background Art

Brown algae contain a variety of sulfated polysaccharides. These sulfated polysaccharides are often generically called sulfated fucans or fucoidins. Their structures may vary depending on the algae from which they derive. For example, sulfated polysaccharides extracted from *Fucus vesiculosus, Kjellmaniella crassifolia, Laminaria japonica*, *Cladosiphon okamuranus, Nemacystus decipiens* and sporophyll of *Undaria pinnatifida* have structures different from each other (see, for example, T. Sakai et al., Bioscience To Industry, June, 2002, 60(6):377-380). In general, a sulfated polysaccharide fraction prepared from a single alga contains a mixture of several molecular species of sulfated polysaccharides.

Molecular species of sulfated polysaccharides of which the structures have been determined includes sulfated fucans (see, for example, WO 99/41288), sulfated fucoglucuronomannans (see, for example, WO 96/34004, WO 02/086116) and sulfated fucogalactans (see, for example, WO 00/50464) and sulfated glucuronofucans (see, for example, WO 01/81560). Sulfated polysaccharides generally have some biological activities in many cases. For example, a sulfated fucan fraction has been reported to have a strong anticoagulant activity (see, for example, WO 99/41288), and a sulfated fucoglucuronomannan fraction has been reported to have an apoptosis-inducing activity against tumor cells (see, for example, WO 97/26896). Thus, attempts have been made to develop sulfated polysaccharides as pharmaceuticals.

If a sulfated polysaccharide is to be developed as a pharmaceutical, it is necessary to determine its structure. It would be very advantageous if an enzyme that degrades the sulfated polysaccharide is used to determine the structure. However, no enzyme that degrades a sulfated polysaccharide from a brown alga is commercially available. In addition, an enzyme that specifically degrades the sulfated polysaccharide of which the structure is to be determined is required. This is because sulfated polysaccharides from brown algae may vary depending on the species of the algae.

A sulfated fucan derived from *Fucus vesiculosus* has been reported to have an anticoagulant activity, an activity of inhibiting a chlamydia from colonizing on uterine epidermal cells, an activity of suppressing an allergic reaction, an activity of suppressing rejection of a transplanted organ and the like (see, for example, Japanese Patent No. 3042543). The structures of sulfated fucans derived from *Fucus vesiculosus* and *Ascophyllum nodosum* have been studied in order to elucidate the relationship between the activities and the structures. For example, average values for the structures have been proposed based on physicochemical analyses by elucidating the structures of a part of oligosaccharides prepared by acid hydrolysis (Lionel Chevolot et al., Carbohydrate Research, 2001, 330:529-535). A structural formula of a sulfated fucan in which sulfate groups are attached to carbons at 2-position and 3-position of the saccharide at the non-reducing end is disclosed in the literature. The oligosaccharide fraction subjected to the structural determination corresponds to about 2% of the entire structure. The oligosaccharide fraction is a mixture of oligosaccharides having various molecular weights. The saccharides at the reducing ends and the non-reducing ends are not uniform. Thus, it has not been proven if the proposed structure is the structure of the main chain of the entire fucoidan. In addition, it is difficult to separate a structurally homogeneous oligosaccharide because the production efficiency is low.

A sulfated fucan fraction prepared from *Fucus vesiculosus* or *Ascophyllum nodosum* contains a mixture of several molecular species of sulfated polysaccharides. In general, sulfated polysaccharides other than the molecular species responsible for the biological activity of interest are unnecessary. In some cases, such unnecessary molecular species may just elicit a side effect.

Capability of reproducible preparation of a structurally homogeneous sulfated fucan oligosaccharide derived from *Fucus vesiculosus* or *Ascophyllum nodosum* is very useful for elucidation of the relationship between the biological activity and the structure. For example, an enzyme that degrades a sulfated fucan contained in a sulfated polysaccharide fraction derived from a brown alga to generate oligosaccharides is known (see, for example, WO 99/41288). This enzyme acts well on a sulfated fucan derived from a brown alga of the order Laminariales to generate sulfated fucan oligosaccharides. However, it does not act on a sulfated fucan derived from a brown alga of the order Fucales (e.g., *Fucus vesiculosus* or *Ascophyllum nodosum*). Furthermore, the sulfated fucoglucuronomannan-degrading enzymes as described in WO 96/34004 and WO 02/086116, and the sulfated fucogalactan-degrading enzyme as described in WO 00/50464 do not act on a sulfated fucan derived from a brown alga of the order Fucales (e.g., *Fucus vesiculosus* or *Ascophyllum nodosum*).

Objects of Invention

As described above, an enzyme that specifically degrades each molecular species contained in a sulfated polysaccharide fraction derived from a brown alga of the order Fucales, an enzymatically produced and structurally homogeneous oligosaccharide, and their production methods have been desired.

Thus, the main object of the present invention is to provide an enzyme that degrades a sulfated fucan derived from a brown alga of the order Fucales which is useful for glycotechnology, a method for producing the enzyme, an oligosaccharide that is obtainable by allowing the enzyme to act on a sulfated fucan, and a method for producing the oligosaccharide.

Summary of Invention

As a result of intensive studies, the present inventors have found that a bacterial strain belonging to the genus *Fucophilus, Fucophilus fucoidanolyticus* SI-1234, produces a novel sulfated fucan-degrading enzyme, and found a method for producing the enzyme. The present inventors have also found that a novel structurally homogeneous sulfated fucan oligosaccharide from a sulfated polysaccharide fraction derived from a brown alga of the order Fucales can be produced utilizing the enzyme. Thus, the present invention has been completed.

The first aspect of the present invention relates to a sulfated fucan-degrading enzyme which is obtainable from a culture of a bacterium of the genus *Fucophilus.*

According to the first aspect, the enzyme can be collected from a culture of a bacterium of the genus *Fucophilus* capable of producing a sulfated fucan-degrading enzyme.

According to the first aspect, examples of the sulfated fucan-degrading enzymes include, but are not limited to, an endo-type α-(1-4)L-fucosidase, an exo-type L-fucosidase, an enzyme A that converts a sulfated fucan into smaller molecules, a sulfated fucan deacetylase and any combinations thereof.

The endo-type α-(1-4)L-fucosidase is exemplified by one having the following physical and chemical properties:

(I) acting on a sulfated fucan oligosaccharide of formula (V) to cleave an α-(1-4)L-fucosyl bond in an endo-type manner:

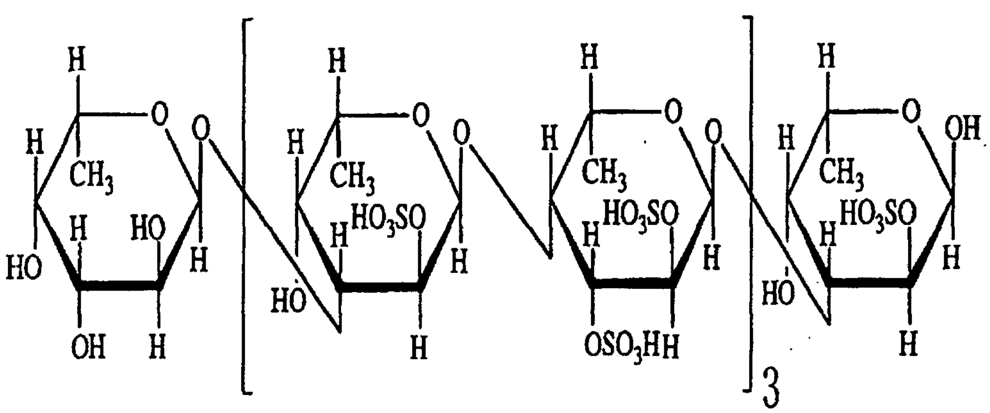

(V)

(II) having an optimal pH of about 6 to 8;
(III) having an optimal temperature of about 10 to 40°C; and
(IV) having a molecular weight of about 110,000 to 130,000 as determined by gel filtration.

The exo-type L-fucosidase has the following physical and chemical properties:

(I) acting on a sulfated fucan to cleave a L-fucose residue in an exo-type manner.

The physical and chemical properties of the enzyme A that converts a sulfated fucan into smaller molecules are as follows:

(I) acting on a sulfated fucan to cleave a sulfate group and release sulfuric acid (sulfated fucan sulfatase); promoting

conversion of a sulfated fucan into smaller molecules.

The physical and chemical properties of the sulfated fucan deacetylase are as follows:

(I) acting on a sulfated fucan to release acetic acid; promoting conversion of a sulfated fucan into smaller molecules.

The second aspect of the present invention relates to a sulfated fucan oligosaccharide which is obtainable by allowing the sulfated fucan-degrading enzyme of the first aspect to act on a sulfated polysaccharide derived from a brown alga.

The sulfated fucan oligosaccharide of the second aspect can be prepared according to a method for producing a sulfated fucan oligosaccharide, the method comprising allowing the sulfated fucan-degrading enzyme of the first aspect to act on a sulfated polysaccharide derived from a brown alga, and collecting a sulfated fucan oligosaccharide.

The third aspect of the present invention relates to a saccharide of general formula (I) or a salt thereof:

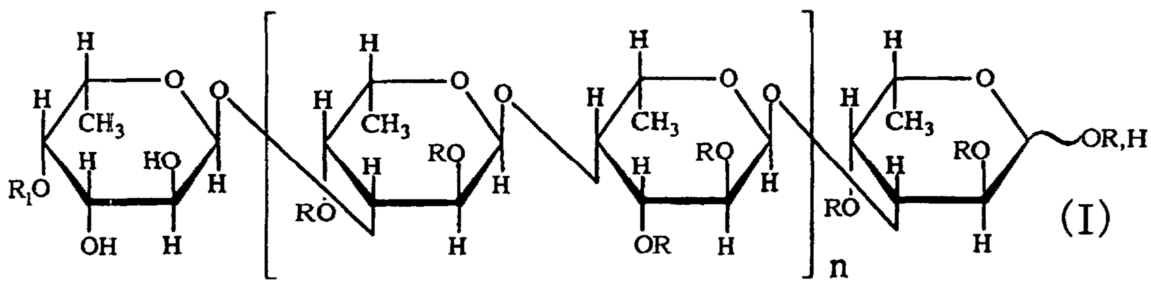

(I)

wherein R is H, $SO_3H$, $CH_3CO$ or general formula (VII); $R_1$ is H, $SO_3H$ or $CH_3CO$; at least one of Rs and $R_1$ is $SO_3H$; n is an integer of 1 or more:

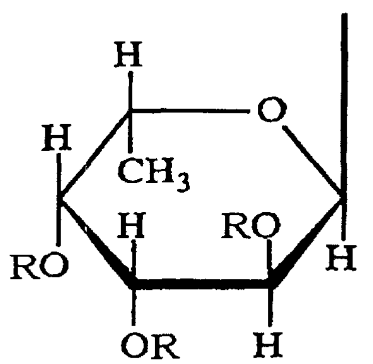

(VII)

wherein R is H, $SO_3H$ or $CH_3CO$.

The fourth aspect of the present invention relates to a sulfated fucan oligosaccharide of general formula (I) or a salt thereof:

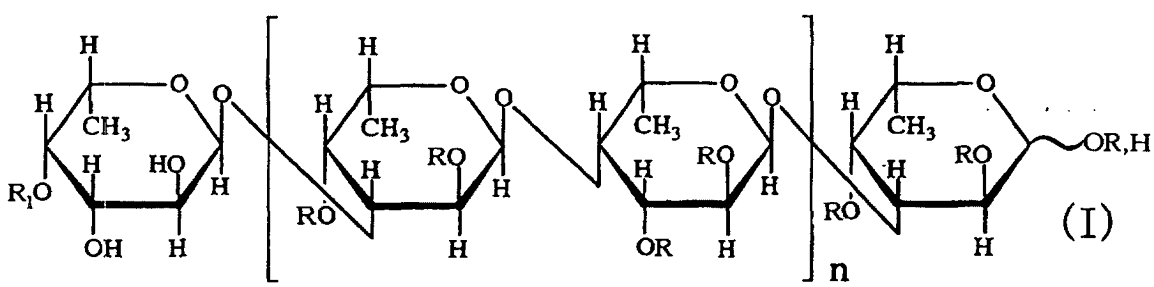

(I)

wherein R is H, $SO_3H$, $CH_3CO$ or general formula (VII); $R_1$ is H, $SO_3H$ or $CH_3CO$; at least one of Rs and $R_1$ is $SO_3H$; n is an integer of 1 to 4:

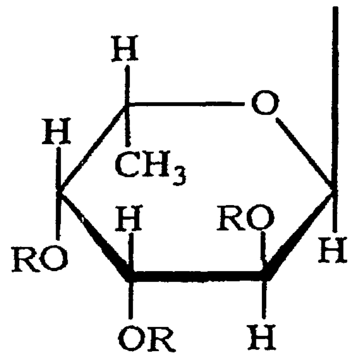
(VII)

wherein R is H, $SO_3H$ or $CH_3CO$.

The fifth aspect of the present invention relates to a production method, the method comprising allowing the sulfated fucan-degrading enzyme of the first aspect to act on a sulfated polysaccharide derived from a brown alga of the order Fucales to produce a sulfated fucan oligosaccharide of general formula (VIII):

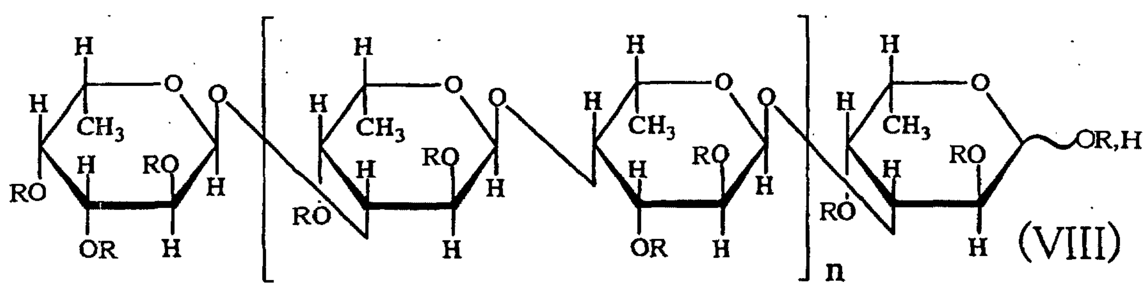
(VIII)

wherein R is H, $SO_3H$, $CH_3CO$ or general formula (VII); at least one of Rs is $SO_3H$; n is an integer of 1 or more:

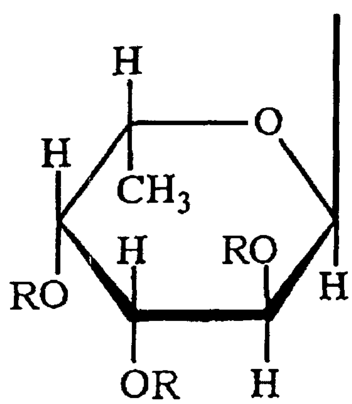
(VII)

wherein R is H, $SO_3H$ or $CH_3CO$.

Brief Description of Drawings

Figure 1: a graph which illustrates the relationship between the pH and the relative activity (%) of the sulfated

fucan-degrading enzyme according to the present invention.

Figure 2: a graph which illustrates the relationship between the temperature (°C) and the relative activity (%) of the sulfated fucan-degrading enzyme according to the present invention.

Figure 3: a figure which illustrates the $^{1}$H-NMR spectrum of the sulfated fucan oligosaccharide 1-(1) according to the present invention.

Figure 4: a figure which illustrates the $^{13}$C-NMR spectrum of the sulfated fucan oligosaccharide 1-(1) according to the present invention.

Figure 5: a figure which illustrates the mass spectrum of the sulfated fucan oligosaccharide 1-(1) according to the present invention.

Figure 6: a figure which illustrates the $^{1}$H-NMR spectrum of the sulfated fucan oligosaccharide 1-(2) according to the present invention.

Figure 7: a figure which illustrates the $^{13}$C-NMR spectrum of the sulfated fucan oligosaccharide 1-(2) according to the present invention.

Figure 8: a figure which illustrates the mass spectrum of the sulfated fucan oligosaccharide 1-(2) according to the present invention.

Figure 9: a figure which illustrates the $^{1}$H-NMR spectrum of the sulfated fucan oligosaccharide 1-(3) according to the present invention.

Figure 10: a figure which illustrates the $^{13}$C-NMR spectrum of the sulfated fucan oligosaccharide 1-(3) according to the present invention.

Figure 11: a figure which illustrates the mass spectrum of the sulfated fucan oligosaccharide 1-(3) according to the present invention.

Figure 12: a figure which illustrates the $^{1}$H-NMR spectrum of the sulfated fucan oligosaccharide 1-(4) according to the present invention.

Figure 13: a figure which illustrates the $^{13}$C-NMR spectrum of the sulfated fucan oligosaccharide 1-(4) according to the present invention.

Figure 14: a figure which illustrates the mass spectrum of the sulfated fucan oligosaccharide 1-(4) according to the present invention.

Figure 15: a figure which illustrates the $^{1}$H-NMR spectrum of the sulfated fucan oligosaccharide 1-(5) according to the present invention.

Figure 16: a figure which illustrates the $^{13}$C-NMR spectrum of the sulfated fucan oligosaccharide 1-(5) according to the present invention.

Figure 17: a figure which illustrates the mass spectrum of the sulfated fucan oligosaccharide 1-(5) according to the present invention.

Figure 18: a graph which illustrates the relationship between the pH and the relative activity (%) of the endo-type $\alpha$-(1-4)L-fucosidase according to the present invention.

Figure 19: a graph which illustrates the relationship between the temperature (°C) and the relative activity (%) of the endo-type $\alpha$-(1-4)L-fucosidase according to the present invention.

Detailed Description of the Invention

Hereinafter, the present invention will be described in detail.

Although it is not intended to limit the present invention, for example, a sulfated fucan derived from a brown alga of the order Fucales (e.g., *Fucus vesiculosus* or *Ascophyllum nodosum*) can be used as a raw material for obtaining the saccharide of the present invention. The brown alga of the order Fucales is preferable as a raw material because a high efficiency of producing the saccharide of the present invention can be achieved with it.

The sulfated fucan-degrading enzyme of the present invention is an enzyme that acts on a sulfated fucan derived from a brown alga to produce an oligosaccharide having fucose at the reducing end.

The sulfated fucan oligosaccharide of the present invention is an oligosacchairde which is obtainable by allowing the sulfated fucan-degrading enzyme of the present invention to act on a sulfated fucan and which has fucose at the reducing end.

A sulfated polysaccharide fraction is first obtained by extracting a brown alga with an aqueous solvent in order to produce the sulfated fucan used according to the present invention. In this case, it is preferable to obtain the fraction at pH 4-9 at a temperature of 100°C or below in order to prevent the conversion of the sulfated fucan into smaller molecules. Furthermore, amino acids, small molecule pigments or the like in the sulfated polysaccharide fraction can be efficiently removed by ultrafiltration. Active carbon treatment or the like is effective for the removal of hydrophobic substances.

A sulfated polysaccharide fraction from a brown alga can be obtained as described above. Any one selected from such a sulfated polysaccharide fraction, a sulfated fucan fraction (a sulfated fucan-containing fraction), and a

sulfated fucan can be used as a substrate for the sulfated fucan-degrading enzyme of the present invention. A more highly pure sulfated fucan can be obtained by separating the fraction using an anion exchange column. Both the sulfated polysaccharide fraction and the sulfated. fucan purified using an anion exchange column can be used as substrates for activity measurements upon purification of the sulfated fucan-degrading enzyme of the present invention and as raw materials for production of sulfated fucan oligosaccharides.

There is no specific limitation concerning the bacterium used for producing the sulfated fucan-degrading enzyme of the present invention as long as it is a bacterium that produces the sulfated fucan-degrading enzyme. For example, *Fucophilus fucoidanolyticus* SI-1234 may be used.

*Fucophilus fucoidanolyticus* SI-1234 is a bacterium newly obtained by the present inventors by screening from a sea cucumber intestine. Its bacteriological properties are as follows.

a. Morphological properties:

Coccus of 1.2 to 1.6 μm in diameter
Spore: no
Gram staining: negative

b. Physiological properties:

(1) Growth temperature: 25°C
(2) Attitude to oxygen: aerobic
(3) Catalase: positive
(4) Oxidase: negative
(5) Salt requirements:

Growth in 0% salt medium: negative
Growth in 1% salt medium: negative
Growth in seawater medium: positive

(6) Quinones: menaquinone 7
(7) GC content of intracellular DNA: 52%
(8) OF-test: not generating acid
(9) Colony color: not generating characteristic colony color
(10) Motility: negative
(11) Gliding: negative
(12) Flagellum: no

This strain is classified into Group 4 (Gram-negative aerobic rods and cocci) according to the basic classification as described in Bergey's Manual of Determinative Bacteriology, 9th ed. (1994). However, this strain is significantly different from bacteria belonging to Group 4 in that it has menaquinone 7 in its electron transport chain and the GC content is 52%.

Then, the nucleotide sequence of the 16S rRNA-encoding DNA of the strain was determined. Comparison of homologies with sequences of known bacteria revealed that there was no known strain that exhibited a high homology over the entire region (about 1500 nucleotides) of the 16S rRNA-encoding DNA. If the homology between entire sequences of 16S rRNA-encoding DNAs from two bacteria is 90% or less, they do not belong to the same genus. A phylogenetic tree prepared based on sequences of 16S rRNA-encoding DNAs registered in a DNA database revealed that all the bacteria related to this strain belonged to Verrucomicrobia. Thus, the present inventors concluded based on the genetic classification that this strain is a bacterium belonging to a new genus of Verrucomicrobia, and designated it as *Fucophilus fucoidanolyticus* SI-1234.

This strain was indicated as *Fucophilus fucoidanolyticus* SI-1234 and deposited at International Patent Organism Depositary, National Institute of Advanced Science and Technology (AIST Tsukuba Central 6, 1-1, Higashi 1-Chome, Tsukuba, Ibaraki 305-8566, Japan) under accession number FERM P-17517 on August 18, 1999 (date of original deposit), and deposited at International Patent Organism Depositary, National Institute of Advanced Science and Technology under Budapest Treaty under accession number FERM BP-7495 on March 7, 2001 (date of request for transmission to international depositary authority).

The sequence of 16S rRNA-encoding DNA of this strain is shown in SEQ ID NO:1. The sulfated fucan-degrading enzymes of the present invention include a sulfated fucan-degrading enzyme obtained from a bacterium that is determined to belong to the genus to which *Fucophilus fucoidanolyticus* SI-1234 belongs based on the sequence

of 16S rRNA-encoding DNA. Bacteria determined to belong to the genus to which *Fucophilus fucoidanolyticus* SI-1234 belongs based on the bacteriological classification or based on the sequences of 16S rRNA-encoding DNAs are determined as bacteria belonging to the genus *Fucophilus*.

Any nutrient source can be added to a medium for culturing a bacterium that produces the sulfated fucan-degrading enzyme of the present invention as long as it is utilized by the microorganism to produce the enzyme in the presence of the nutrient source. For example, a sulfated fucan, an alga (e.g., *Fucus vesiculosus* or *Ascophyllum nodosum*), alginic acid, laminaran, fucose, glucose, mannitol, glycerol, saccharose, maltose, starch or the like can be utilized as a carbon source. Yeast extract, peptone, casamino acid, corn steep liquor, meat extract, defatted soybean, ammonium sulfate, ammonium chloride, urea, uric acid or the like is suitable as a nitrogen source. In addition, a chloride, a phosphate or a sulfate of sodium, potassium, magnesium, calcium, zinc or the like may be added. In general, a microorganism obtained from seawater can be very readily grown in seawater or commercially available artificial seawater.

The culture conditions are determined such that the productivities of the sulfated fucan-degrading enzyme of the present invention become maximal depending on the microorganism to be used, the composition of the medium and the like. In general, the maximal productivity of the sulfated fucan-degrading enzyme of the present invention is achieved by culturing with aeration and stirring at a culture temperature of 15 to 30°C at medium pH of 5 to 9 for 5 to 72 hours. The sulfated fucan-degrading enzyme of the present invention can be obtained from cells and a culture supernatant separated from each other by centrifugation after culturing.

A cell-free extract is obtained by culturing *Fucophilus fucoidanolyticus* SI-1234 in an appropriate medium, collecting the cells, and disrupting the cells using a conventional means of cell disruption (e.g., sonication). The sulfated fucan-degrading enzyme can be purified from the extract using a conventional means of purification. The sulfated fucan-degrading enzyme of the present invention can be obtained in a purified form using, for example, salting out, ion exchange column chromatography, hydrophobic column chromatography, gel filtration or the like. Furthermore, a means of purification similar to that used for the purification of the intracellular enzyme can be used to purify the sulfated fucan-degrading enzyme of the present invention from the culture supernatant which also contains the enzyme. The sulfated fucan-degrading enzymes of the present invention include at least the following four enzymes: an endo-type α-(1-4)L-fucosidase, an exo-type L-fucosidase, an enzyme A that converts a sulfated fucan into smaller molecules and a sulfated fucan deacetylase.

The physical and chemical properties of the sulfated fucan-degrading enzyme of the present invention are as follows:

(I) acting on a sulfated fucan to produce an oligosaccharide having fucose at the reducing end;
(II) having an optimal pH of about 7.0 to 8.5 (Figure 1, a graph illustrating the relationship between the reaction pH and the relative activity of the enzyme, in which the longitudinal axis represents the relative activity (%) and the horizontal axis represents the pH); and
(III) having an optimal temperature of about 30 to 40°C (Figure 2, a graph which illustrates the relationship between the reaction temperature and the relative activity of the enzyme, in which the longitudinal axis represents the relative activity (%) and the horizontal axis represents the temperature (°C)).

The physical and chemical properties of the endo-type α-(1-4)L-fucosidase as an example of the sulfated fucan-degrading enzyme of the present invention are as follows:

(I) acting on a sulfated fucan oligosaccharide of formula (V) to cleave an α-(1-4)L-fucosyl bond in an endo-type manner:

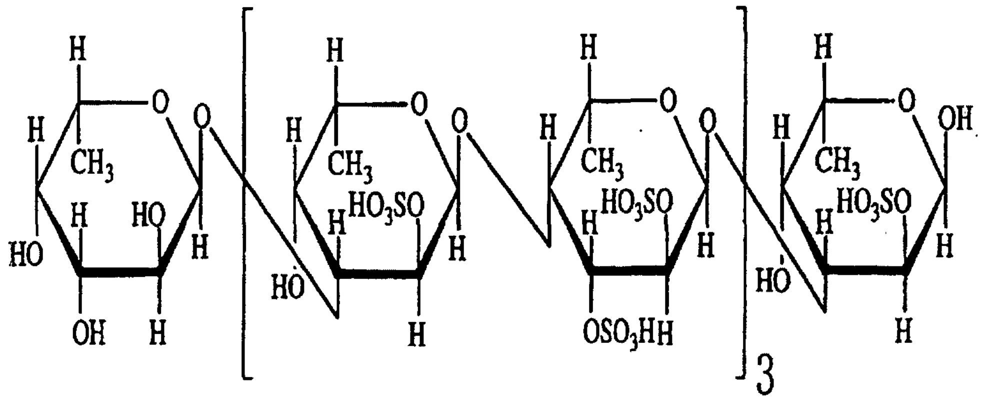

(V)

(II) having an optimal pH of about 6 to 8 (Figure 18, a graph illustrating the relationship between the reaction pH and the relative activity of the enzyme, in which the longitudinal axis represents the relative activity (%) and the horizontal axis represents the pH);

(III) having an optimal temperature of about 10 to 40°C (Figure 19, a graph which illustrates the relationship between the reaction temperature and the relative activity of the enzyme, in which the longitudinal axis represents the relative activity (%) and the horizontal axis represents the temperature (°C)); and

(IV) having a molecular weight of about 110,000 to 130,000 as determined by gel filtration.

The physical and chemical properties of the exo-type L-fucosidase as an example of the sulfated fucan-degrading enzyme of the present invention are_as follows:

(I) acting on a sulfated fucan to cleave a L-fucose residue in an exo-type manner.

The physical and chemical properties of the enzyme A that converts a sulfated fucan into smaller molecules as an example of the sulfated fucan-degrading enzyme of the present invention are as follows:

(I) acting on a sulfated fucan to cleave a sulfate group and release sulfuric acid (sulfated fucan sulfatase); promoting conversion of a sulfated fucan into smaller molecules.

The physical and chemical properties of the sulfated fucan deacetylase as an example of the sulfated fucan-degrading enzyme of the present invention are as follows:

(I) acting on a sulfated fucan to release acetic acid; promoting conversion of a sulfated fucan into smaller molecules.

The sulfated fucan-degrading enzyme of the present invention can be identified by measuring an activity of degrading a sulfated fucan. A cell-free extract from a producer strain or an enzyme solution obtained after purification using various column chromatographies may be used for the identification.

*Fucophilus fucoidanolyticus* SI-1234 is a microorganism that utilizes a sulfated fucan, and produces the sulfated fucan-degrading enzyme of the present invention inside or outside the cell to degrade the sulfated fucan.

The sulfated fucan oligosaccharide of the present invention can be prepared by allowing the sulfated fucan-degrading enzyme of the present invention to act on a sulfated fucan or a sulfated fucan-containing material. For example, a partially purified preparation of sulfated fucan, a sulfated polysaccharide fraction derived from a brown alga, a product obtained by extracting a brown alga with an aqueous solvent, or a brown alga itself can be preferably used as a sulfated fucan-containing material.

For preparing the sulfated fucan oligosaccharide of the present invention, a sulfated fucan or a sulfated fucan-containing material may be dissolved according to a conventional method. The sulfated fucan or the sulfated fucan-containing material may be dissolved in the solution at the maximal concentration. However, the concentration is usually selected taking its operationality, the amount of the sulfated fucan-degrading enzyme of the present invention to be used in a reaction and the like into consideration. The solvent for the sulfated fucan may be selected from water, buffers and the like depending on the objects. Usually, the pH of the solution is nearly neutral. The enzymatic reaction is usually carried out at about 30°C. The molecular weight of the sulfated fucan oligosaccharide can be controlled by adjusting the amount of the sulfated fucan-degrading enzyme of the present invention used in the reaction, the composition of

the reaction mixture, the reaction time and the like. The sulfated fucan oligosaccharide of the present invention having more homogeneous molecular weight distribution can be prepared by subjecting the sulfated fucan oligosaccharide of the present invention obtained as described above to molecular weight fractionation or fractionation using an anion exchange column. A conventional method of molecular weight fractionation (e.g., gel filtration or ultrafiltration) may be used. Optionally, the smaller molecules may be further purified using ion exchange resin treatment, active carbon treatment or the like, or they may be desalted, sterilized or lyophilized. Thus, the sulfated fucan oligosaccharide of the present invention having a structure so homogeneous that one can determine the structure by NMR analysis as described below can be obtained.

The sulfated fucan oligosaccharides of the present invention have sulfate groups within the molecules, which groups react with various bases to form salts. The sulfated fucan oligosaccharide of the present invention is stable when it is in a form of salt. It is usually provided in a form of sodium and/or potassium and/or calcium salt. The sulfated fucan oligosaccharide of the present invention in a free form can be derived from a salt thereof by utilizing cation exchange resin such as Dowex 50W. Optionally, it can be subjected to conventional salt-exchange to convert it into any one of various desirable salts.

Pharmaceutically acceptable salts can be used as the salts of the sulfated fucan oligosaccharide of the present invention. Examples of the salts include salts with alkaline metals (e.g., sodium and potassium) and alkaline earth metals (e.g., calcium, magnesium and zinc) as well as ammonium salts.

The saccharide or the sulfated fucan oligosaccharide of the present invention is characterized by the structure in which hydroxyl groups are attached to carbons at 2-position and 3-position of the saccharide at the non-reducing end. In addition, an acetyl group or a fucosyl group may be attached to the saccharide or the sulfated fucan oligosaccharide of the present invention.

The sulfated fucan-degrading enzyme of the present invention can be used for structural analysis of a sulfated fucan because it converts a sulfated fucan into smaller molecules. Additionally, the sulfated fucan oligosaccharide of the present invention can be used as a reagent for glycotechnology. For example, a substance that is very useful as a reagent for glycotechnology (e.g., that can be used as a substrate for various sulfated fucan-degrading enzymes) can be provided by subjecting the oligosaccharide to amination with 2-aminopyridine (PA-labeling) according to the method as described in JP-B 5-65108 to prepare a PA-labeled oligosaccharide as described below. Furthermore, the sulfated fucan oligosaccharide of the present invention can be used as an antigen to produce an antibody. Such an antibody can be used to identify the structure of a sulfated polysaccharide.

## Examples

The following examples further illustrate the present invention in detail but are not to be construed to limit the scope thereof.

### Referential Example 1: Preparation of sulfated polysaccharide fraction from *Fucus vesiculosus*

1 kg of dried *Fucus vesiculosus* was disrupted and suspended in 10 L of 80% ethanol. The suspension was stirred at 25°C for 3 hours, filtered and washed to obtain a residue. The residue was suspended in 30 L of 30 mM phosphate buffer (pH 6.5) containing 100 mM sodium chloride. The suspension was treated at 95°C for 2 hours and then cooled to 30°C. 100 g of active carbon, 3000 U of alginate lyase K (Nagase Biochemicals) and 3.75 L of ethanol were added thereto. The mixture was stirred for 24 hours, and then centrifuged to obtain a supernatant. The supernatant was concentrated to 4 L using an ultrafiltration device equipped with hollow fibers with exclusion molecular weight of 100,000 and subjected to solvent exchange for 100 mM sodium chloride. The solution was cooled to 4°C, and the pH was adjusted to 2.0 with 0.5 N hydrochloric acid. The formed precipitate was removed by centrifugation to obtain a supernatant. The pH of the supernatant was adjusted to 8.0 with 1 N sodium hydroxide. The supernatant was concentrated to 2 L using the above-mentioned ultrafiltration device and subjected to solvent exchange for 20 mM sodium chloride. Insoluble substances were removed by centrifugation. The supernatant was then lyophilized to obtain 80 g of a sulfated polysaccharide fraction from *Fucus vesiculosus*.

### Referential Example 2: Preparation of sulfated polysaccharide fraction from *Ascophyllum nodosum*

100 g of a sulfated polysaccharide fraction from *Ascophyllum nodosum* was obtained from 1 kg of commercially available *Ascophyllum nodosum* powder according to the method as described in Referential Example 1.

### Referential Example 3: Preparation of sulfated polysaccharide fraction from *Kjellmaniella crassifolia*

38 g of a sulfated polysaccharide fraction from *Kjellmaniella crassifolia* was obtained according to the method

as described in Referential Example 1 from 1 kg of chips prepared from commercially available dried *Kjellmaniella crassifolia* by disruption using a cutter mill (Masuko Sangyo).

Referential Example 4: Preparation of sulfated fucan fraction from *Fucus vesiculosus*

7 g of the sulfated polysaccharide fraction from *Fucus vesiculosus* as described in Referential Example 1 was dissolved in 700 ml of 20 mM imidazole-hydrochloride buffer (pH 6.0) containing 100 mM sodium chloride and loaded onto a 5-L DEAE-Cellulofine A-800 equilibrated with the same buffer. After loading the sample and washing with 10 L of the same buffer, elution was carried out with a gradient of 100 to 1600 mM sodium chloride to collect fractions each containing 500 ml of the eluate. The total sugar content and the total uronic acid content of each fraction were measured according to the phenol-sulfuric acid method and the carbazole-sulfuric acid method, respectively. A fraction eluted with 700 to 800 mM sodium chloride was concentrated and desalted by ultrafiltration (exclusion molecular weight of 100,000), and lyophilized to obtain 1.0 g of a sulfated fucan fraction from *Fucus vesiculosus*.

Referential Example 5: Preparation of sulfated fucan fraction from *Ascophyllum nodosum*

1.1 g of a sulfated fucan fraction from *Ascophyllum nodosum* was obtained from 7 g of the sulfated polysaccharide fraction from Ascophyllum nodosum as described in Referential Example 2 according to the method as described in Referential Example 4.

Referential Example 6: Method for measuring activity of sulfated fucan-degrading enzyme

The sulfated fucan-degrading enzyme of the present invention acts on a sulfated fucan to convert the sulfated fucan into smaller molecules. The activity of the sulfated fucan-degrading enzyme was measured as follows utilizing this action. The sulfated fucan-degrading enzyme of the present invention acts on sulfated fucans derived from *Fucus vesiculosus* and *Ascophyllum nodosum*. Among these, the sulfated fucan derived from *Fucus vesiculosus* was used as a substrate for the activity measurements.

Briefly, 4.8 μl of a 2.5% solution of the sulfated fucan fraction from *Fucus vesiculosus* as described in Referential Example 4, 60 μl of 50 mM imidazole-hydrochloride buffer (pH 7.8), 6 μl of 1 M calcium chloride, 9 μl of 4 M sodium chloride, 28.2 μl of water and 12 μl of a solution of the sulfated fucan-degrading enzyme were mixed together. After reacting at 30°C for 3 hours, the reaction mixture was treated at 100°C for 10 minutes and analyzed using HPLC. As controls, a reaction mixture obtained by a reaction in which the solvent for the sulfated fucan-degrading enzyme was used in place of the enzyme solution and a reaction mixture obtained by a reaction in which water was used in place of the sulfated fucan fraction were similarly analyzed. The analysis using HPLC was carried out as follows:

Column: Shodex SB-806HQ;
Column temperature: 25°C;
Eluent :50 mM sodium chloride containing 5 mM sodium azide;
Flow rate: 1 ml/minute;
Detection: differential refractive index detector.

One unit of a sulfated fucan-degrading enzyme activity is defined as an amount of an enzyme that cleaves 1 μmole of fucosyl bond in 1 minute in the above-mentioned reaction system. The amount of cleaved fucosyl bond was calculated according to the following equation:

$$Fd \times (Sm / Pm - 1)/(Sm \times 180 \times 0.01) = U/ml$$

Fd: the amount of the sulfated fucan used for reaction (μg);
Sm: the average molecular weight of the sulfated fucan as a substrate;
Pm: the average molecular weight of the sulfated fucan after reaction;
180: the reaction time (minutes); and
0.01: the volume of the enzyme solution (ml).

The amount of protein was determined by measuring the absorbance of the enzyme solution at 280 nm. The calculation was carried out assuming the absorbance of a solution containing.a protein at a concentration of 1 mg/ml as 1.0.

Example 1: Preparation of sulfated fucan-degrading enzyme

*Fucophilus fucoidanolyticus* SI-1234 was inoculated into 600 ml of a medium consisting of artificial seawater (Jamarine Laboratory) containing the sulfated polysaccharide fraction from *Fucus vesiculosus* as described in Referential Example 1 and peptone at concentrations of 0.2% and 1%, respectively, (pH 8.0) which had been autoclaved at 120°C for 20 minutes, and cultured at 24°C for 72 hours to prepare a seed culture. 20 L of a medium consisting of artificial seawater (Jamarine Laboratory) containing the sulfated polysaccharide fraction from *Fucus vesiculosus* as described in Referential Example 1 and peptone at concentrations of 0.2% and 1%, respectively, and antifoaming agent (KM70, Shin-Etsu Chemical) (pH 8.0) was placed in a 30-L jar fermentor and autoclaved at 120°C for 20 minutes. The seed culture was inoculated into the medium and cultured at 24°C for 48 hours with stirring at 125 rpm. After cultivation, the culture was centrifuged to obtain cells and a culture supernatant.

The cells were suspended in 700 ml of 10 mM imidazole-hydrochloride buffer (pH 7.0) containing 250 mM sodium chloride and 10 mM calcium chloride, sonicated and centrifuged to obtain a supernatant. The supernatant was adequately dialyzed against the same buffer and centrifuged to obtain a supernatant as a crude enzyme solution of the sulfated fucan-degrading enzyme.

The sulfated fucan-degrading enzyme activities contained in the culture supernatant and the crude enzyme solution were measured according to the method as described in Referential Example 6. As a result, 0.05 mU and 0.05 mU of the activities were detected for the culture supernatant corresponding to 1 ml of the culture and the cell extract corresponding to 1 ml of the culture, respectively.

The crude enzyme solution of the sulfated fucan-degrading enzyme was subjected to solvent exchange by ultrafiltration for 10 mM imidazole-hydrochloride buffer (pH 7.0) containing 10 mM calcium chloride, 30 mM sodium chloride and 5 mM sodium azide, and loaded onto a 500-ml DEAE-Cellulofine A-800 column equilibrated with the same buffer. After adding the enzyme solution and adequately washing with the same buffer, elution was carried out with a gradient of 30 to 350 mM sodium chloride to collect fractions each containing 51 ml of the eluate.

The sulfated fucan-degrading enzyme activities were measured for the respective fractions. Among active fractions, a fraction eluted with 60 to 140 mM. sodium chloride was designated as a DEAE-1 fraction, and a fraction eluted with 140 to 230 mM sodium chloride was designated as a DEAE-2 fraction

The DEAE-1 fraction was loaded onto a 200-ml Sulfate-Cellulofine (Seikagaku Corporation) equilibrated with 10 mM imidazole-hydrochloride buffer (pH 7.0) containing 10 mM calcium chloride, 100 mM sodium chloride and 5 mM sodium azide. After adding the enzyme solution and adequately washing with the same buffer, elution carried out with a gradient of 100 to 600 mM sodium chloride to collect fractions each containing 20 ml of the eluate.

The sulfated fucan-degrading enzyme activities were measured for the respective fractions. Among active fractions, a fraction eluted with 290 to 370 mM sodium chloride was designated as a DEAE-1S fraction.

The DEAE-2 fraction was loaded onto a 200-ml Sulfate-Cellulofine (Seikagaku Corporation) equilibrated with 10 mM imidazole-hydrochloride buffer (pH 7.0) containing 10 mM calcium chloride, 200 mM sodium chloride and 5 mM sodium azide. After adding the enzyme solution and adequately washing with the same buffer, elution was carried out with a gradient of 200 to 1500 mM sodium chloride to collect fractions each containing 20 ml of the eluate.

The sulfated fucan-degrading enzyme activities were measured for the respective fractions. Among active fractions, a fraction eluted with 240 to 350 mM sodium chloride was designated as a DEAE-2S fraction.

Example 2: Preparation of sulfated fucan oligosaccharide using crude enzyme solution of sulfated fucan-degrading enzyme, and purification and structural analysis thereof

(1) Preparation of substrate

60 g of dried *Fucus vesiculosus* was disrupted and suspended in 1 L of 80% ethanol. The suspension was stirred for 2 hours and filtered. The residue was washed adequately. After two more rounds of the washing step, the washed residue was suspended in 4 L of 20 mM imidazole-hydrochloride buffer (pH 7.0) containing 50 mM calcium chloride, 400 mM sodium chloride and 10% ethanol. The suspension was stirred for 24 hours and centrifuged to obtain a *Fucus vesiculosus* extract.

The *Fucus vesiculosus* extract was subjected to ultrafiltration using an ultrafiltration device equipped with hollow fibers with exclusion molecular weight of 100,000 while adding the extraction buffer to remove low molecular weight substances. The final volume was 320 ml. Precipitates were removed by centrifugation. Thus, a *Fucus vesiculosus* macromolecule fraction was obtained.

(2) Preparation of sulfated fucan oligosaccharides

The whole *Fucus vesiculosus* macromolecule fraction as described in Example 2-(1) was mixed with the

DEAE-1S fraction (corresponding to 1.78 mU of the sulfated fucan-degrading enzyme activity) and the DEAE-2S fraction (corresponding to 1.97 mU of the sulfated fucan-degrading enzyme activity) as described in Example 1. The mixture was reacted at 25°C for 5 days. The mixture was mixed with the DEAE-1S fraction (corresponding to 1.54 mU of the sulfated fucan-degrading enzyme activity) and the DEAE-2S fraction (corresponding to 1.70 mU of the sulfated fucan-degrading enzyme activity) as described in Example 1. The mixture was reacted at 25°C for 13 days.

The reaction mixture was loaded onto an ultrafiltration device equipped with hollow fibers with exclusion molecular weight of 10,000, a fraction of oligosaccharides of molecular weights of 10,000 or less was collected and designated as a sulfated fucan oligosaccharide mixture 1.

(3) Purification of sulfated fucan oligosaccharides

Water was added to the sulfated fucan oligosaccharide mixture 1 obtained in Example 2-(2). Imidazole and sodium chloride were added thereto at final concentrations of 10 mM and 10 mM, respectively. The resulting mixture was loaded onto a 500-ml DEAE-Cellulofine A-800 column equilibrated with 10 mM imidazole-hydrochloride buffer (pH 6.0) containing 10 mM sodium chloride. After washing with 1 L of the same buffer, elution was carried out with a gradient of 10 to 1200 mM sodium chloride to collect fractions each containing 50 ml of the eluate. The total sugar content of each fraction was measured according to the phenol-sulfuric acid method. As a result, the eluted fractions formed five peaks. The fractions in the respective peaks were collected to obtain oligosaccharide fractions 1-(1) to 1-(5).

The oligosaccharide fractions 1-(1) to 1-(3) were concentrated using an evaporator to 40 ml, loaded onto a Cellulofine GCL-25 column (4.1 x 90.5 cm) equilibrated with 10% ethanol and eluted with 10% ethanol for desalting. The desalted oligosaccharide fractions 1-(1) and 1-(2) were lyophilized. Thus, 15 mg of the sulfated fucan oligosaccharide 1-(1) and 75 mg of the sulfated fucan oligosaccharide 1-(2) were obtained.

The desalted oligosaccharide fraction 1-(3) was collected. Imidazole and sodium chloride were added thereto at final concentrations of 10 mM and 200 mM, respectively. The pH was adjusted to 6. The thus obtained oligosaccharide solution was loaded onto a 100-ml DEAE-Cellulofine A-800 column equilibrated with 10 mM imidazole-hydrochloride buffer (pH 6.0) containing 200 mM sodium chloride. After washing with 300 ml of the same buffer, elution was carried out with a gradient of 200 to 700 mM sodium chloride to collect fractions each containing 10 ml of the eluate. The total sugar content of each fraction was measured according to the phenol-sulfuric acid method. As a result, a fraction eluted with salt concentrations of 430 to 480 mM which contained an oligosaccharide having a homogeneous molecular weight was collected. The fraction was concentrated using an evaporator to 40 ml, loaded onto a Cellulofine GCL-25 column (4.1 x 90.5 cm) equilibrated with 10% ethanol and eluted with 10% ethanol for desalting. The desalted oligosaccharide solution was lyophilized. Thus, 35 mg of the sulfated fucan oligosaccharides 1-(3) was obtained.

The conductivity of the oligosaccharide fraction 1-(4) was adjusted by adding water to the same conductivity of 10 mM imidazole-hydrochloride buffer (pH 6) containing 300 mM sodium chloride. The oligosaccharide solution was loaded onto a 50-ml DEAE-Cellulofine A-800 column equilibrated with 10 mM imidazole-hydrochloride buffer (pH 6.0) containing 300 mM sodium chloride. After washing with 100 ml of the same buffer, elution was carried out with a gradient of 300 to 800 mM sodium chloride to collect fractions each containing 4.9 ml of the eluate. The total sugar content of each fraction was measured according to the phenol-sulfuric acid method. As a result, a fraction eluted with salt concentrations of 450 to 630 mM which contained an oligosaccharide having a homogeneous molecular weight was collected. The fraction was concentrated using an evaporator to 40 ml, loaded onto a Cellulofine GCL-25 column (4.1 x 90.5 cm) equilibrated with 10% ethanol and eluted with 10% ethanol for desalting. The desalted oligosaccharide solution was lyophilized. Thus, 112 mg of the sulfated fucan oligosaccharides 1-(4) was obtained.

The conductivity of the oligosaccharide fraction 1-(5) was adjusted by adding water to the same conductivity of 10 mM imidazole-hydrochloride buffer (pH 6) containing 400 mM sodium chloride. The oligosaccharide solution was loaded onto a 100-ml DEAE-Cellulofine A-800 column equilibrated with 10 mM imidazole-hydrochloride buffer (pH 6.0) containing 400 mM sodium chloride. After washing with 200 ml of the same buffer, elution was carried out with a gradient of 400 to 900 mM sodium chloride to collect fractions each containing 10 ml of the eluate. The total sugar content of each fraction was measured according to the phenol-sulfuric acid method. As a result, a fraction eluted with salt concentrations of 640 to 700 mM which contained an oligosaccharide having a homogeneous molecular weight was collected. The fraction was concentrated using an evaporator to 40 ml, loaded onto a Cellulofine GCL-25 column (4.1 x 90.5 cm) equilibrated with 10% ethanol and eluted with 10% ethanol for desalting. The desalted oligosaccharide solution was lyophilized. Thus, 44 mg of the sulfated fucan oligosaccharides 1-(5) was obtained.

(4) Structural analyses

The sulfated fucan oligosaccharides 1-(1) to 1-(5) obtained in Example 2-(3) were fluorescently labeled with 2-aminopyridine, and subjected to analyses of saccharides at the reducing ends and saccharide compositions. As a result, the saccharide at the reducing end was determined to be fucose for each of the sulfated fucan oligosaccharides

1-(1) to 1-(5). Regarding the neutral sugar composition, each oligosaccharide consisted of fucose. Next, determination of the sulfuric acid content (measured according to the turbidinetric method using barium chloride), mass spectrometric analyses using a mass spectrometer API-III (Perkin-Elmer Sciex) and NMR analyses using a nuclear magnetic resonance apparatus JNM $\alpha$-500 (Nippon Denshi) and Digital NMR ADVANCE 600 (Bruker Analytik) were carried out. Samples to be analyzed were subjected to structural analyses after exchange for heavy water according to a conventional method. Bonds of constituting saccharides were analyzed using the HMBC method, a method for $^1$H-detection of heteronuclei. The DQF-COSY method and the HOHAHA method were used for identification in $^1$H-NMR. The HSQC method was used for identification in $^{13}$C-NMR. Physical properties of the sulfated fucan oligosaccharides 1-(1) to 1-(5) are shown below.

(a) Physical properties of the sulfated fucan oligosaccharide 1-(1)

The results for mass spectrometric analysis and identification in NMR analysis are shown below. The $^1$H-NMR spectrum, $^{13}$C-NMR spectrum and mass spectrum are illustrated in Figures 3, 4 and 5, respectively. In Figures 3 and 4, the longitudinal axes represent the signal intensity and the horizontal axes represent the chemical shift value (ppm). In Figure 5, the longitudinal axis represents the relative intensity (%) and the horizontal axis represents the m/z value.
Molecular weight: 842
MS m/z; 431.1, $[M+Na^+-2H^+]^{2-}$; 885.2, $[M+2Na^+-3H^+]^-$
Results of $^1$H-NMR and $^{13}$C-NMR analyses are shown in Tables 1 and 2.

Table 1

| | Chemical shift value (ppm) | |
|---|---|---|
| | $^1$H-NMR Chemical shift value, multiplicity, coupling constant | $^{13}$C-NMR |
| F1-1 | 5.43, d, 4.0 | 91.3 |
| F1-2 | 4.45, m | 74.3 |
| F1-3 | 3.99, dd, 3.5, 10.0 | 73.5 |
| F1-4 | 4.02, d, 3.5 | 69.5 |
| F1-5 | 4.16, q, 6.5 | 66.8 |
| F1-6 | 1.18, d, 6.5 | 16.3 |
| F2-1 | 5.29, d, 3.0 | 95.0 |
| F2-2 | 4.44, m | 76.2 |
| F2-3 | 4.11, m | 68.2 |
| F2-4 | 3.93, d, 3.0 | 83.3 |
| F2-5 | 4.46, m | 68.5 |
| F2-6 | 1.32, d, 6.5 | 16.4 |

Table 2

| | Chemical shift value (ppm) | |
|---|---|---|
| | $^1$H-NMR Chemical shift value, multiplicity, coupling constant | $^{13}$C-NMR |
| F3-1 | 5.21, d, 3.5 | 99.9 |
| F3-2 | 4.51, dd, 3.5, 10.5 | 74.3 |
| F3-3 | 4.13, m | 72.6 |
| F3-4 | 4.04, d, 3.0 | 69.8 |
| F3-5 | 4.38, m | 68.2 |
| F3-6 | 1.18, d, 6.5 | 16.2 |
| F4-1 | 5.00, d, 4.0 | 96.5 |

Table 2 (continued)

| | Chemical shift value (ppm) | |
|---|---|---|
| | $^{1}$H-NMR Chemical shift value, multiplicity, coupling constant | $^{13}$C-NMR |
| F4-2 | 3.73, dd, 4.0, 10.5 | 68.9 |
| F4-3 | 3.90, dd, 3.5, 10.5 | 70.3 |
| F4-4 | 3.77, d, 3.5 | 72.9 |
| F4-5 | 4.41, m | 67.8 |
| F4-6 | 1.15, d, 6.5 | 16.2 |
| Saccharide composition: 4 molecules of L-fucose<br>Sulfate group: 3 molecules | | |

The numbers for peak identification in $^{1}$H-NMR and $^{13}$C-NMR are as indicated in formula (II) below:

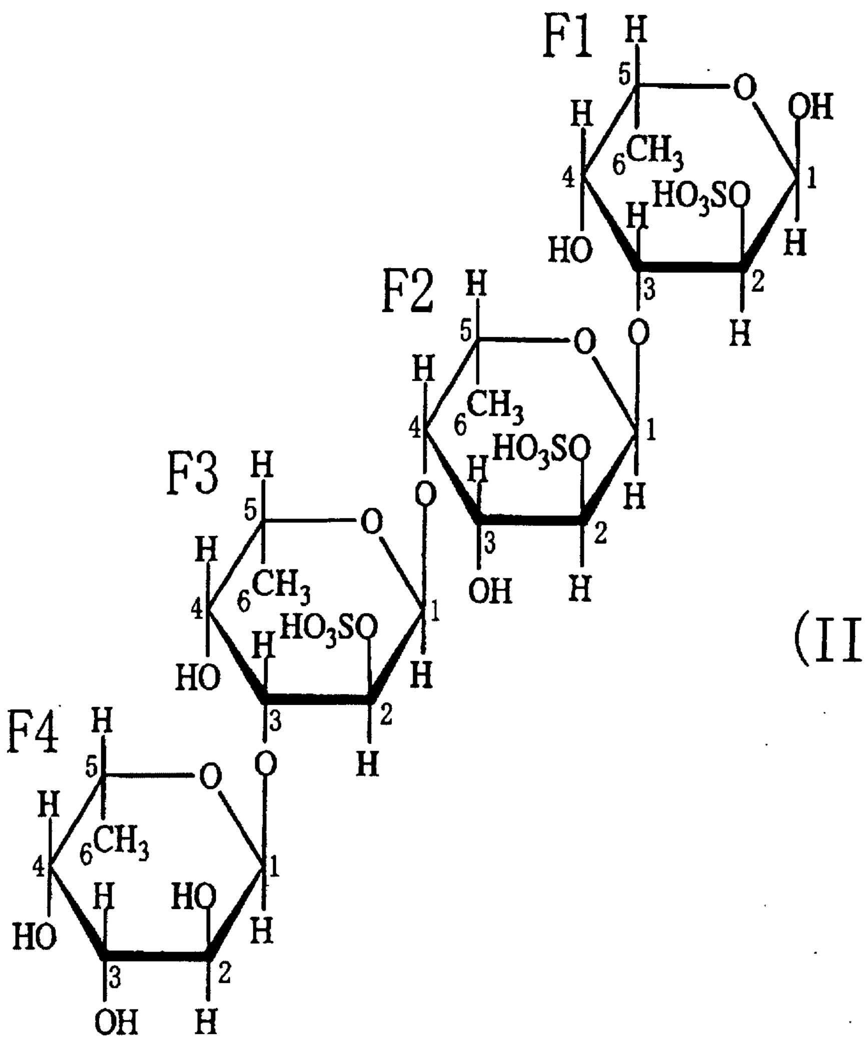

(II)

(b) Physical properties of the sulfated fucan oligosaccharide 1-(2)

The results for mass spectrometric analysis and identification in NMR analysis are shown below. The $^{1}$H-NMR spectrum, $^{13}$C-NMR spectrum and mass spectrum are illustrated in Figures 6, 7 and 8, respectively. In Figures 6 and

7, the longitudinal axes represent the signal intensity and the horizontal axes represent the chemical shift value (ppm). In Figure 8, the longitudinal axis represents the relative intensity (%) and the horizontal axis represents the m/z value.
Molecular weight: 922
MS m/z; 482.1, $[M+2Na^{+}-4H^{+}]^{2-}$; 987.1, $[M+3Na^{+}-4H^{+}]^{-}$

Results of $^{1}H$-NMR and $^{13}C$-NMR analyses are shown in Tables 3 and 4.

Table 3

| | Chemical shift value (ppm) | |
|---|---|---|
| | $^{1}H$-NMR Chemical shift value, multiplicity, coupling constant | $^{13}C$-NMR |
| F1-1 | 5.43, d, 3.5 | 91.4 |
| F1-2 | 4.46, dd, 3.9, 10.4 | 74.2 |
| F1-3 | 3.99, dd, 3.1, 10.4 | 74.64 or 74.68 |
| F1-4 | 4.02, d, 2.5 | 69.99 or 70.02 |
| F1-5 | 4.16, m | 66.8 |
| F1-6 | 1.17, d, 6.5 | 16.3 |
| F2-1 | 5.30, d, 3.8 | 96.0 |
| F2-2 | 4.58, dd, 3.6, 11.0 | 73.3 |
| F2-3 | 4.78, dd, 2.7, 10.9 | 74.64 or 74.68 |
| F2-4 | 4.21, d, 2.9 | 80.0 |
| F2-5 | 4.49, m | 68.7 |
| F2-6 | 1.32, d, 6.5 | 16.4 |

Table 4

| | Chemical shift value (ppm) | |
|---|---|---|
| | $^{1}H$-NMR Chemical shift value, multiplicity, coupling constant | $^{13}C$-NMR |
| F3-1 | 5.21, d, 3.4 | 99.5 |
| F3-2 | 4.49, m | 74.2 |
| F3-3 | 4.14, m | 72.5 |
| F3-4 | 4.02, d, 2.5 | 69.99 or 70.02 |
| F3-5 | 4.39, m | 68.0 |
| F3-6 | 1.22, d, 6.0 | 16.18 |
| F4-1 | 5.00, d, 4.0 | 96.4 |
| F4-2 | 3.72, dd, 4.0, 10.0 | 69.0 |
| F4-3 | 3.90, dd, 3.0, 10.0 | 70.3 |
| F4-4 | 3.75, d, 3.0 | 72.9 |
| F4-5 | 4.41, m | 67.7 |
| F4-6 | 1.14, d, 6.5 | 16.17 |
| Saccharide composition: 4 molecules of L-fucose<br>Sulfate group: 4 molecules | | |

The numbers for peak identification in $^{1}H$-NMR and $^{13}C$-NMR are as indicated in formula (III) below:

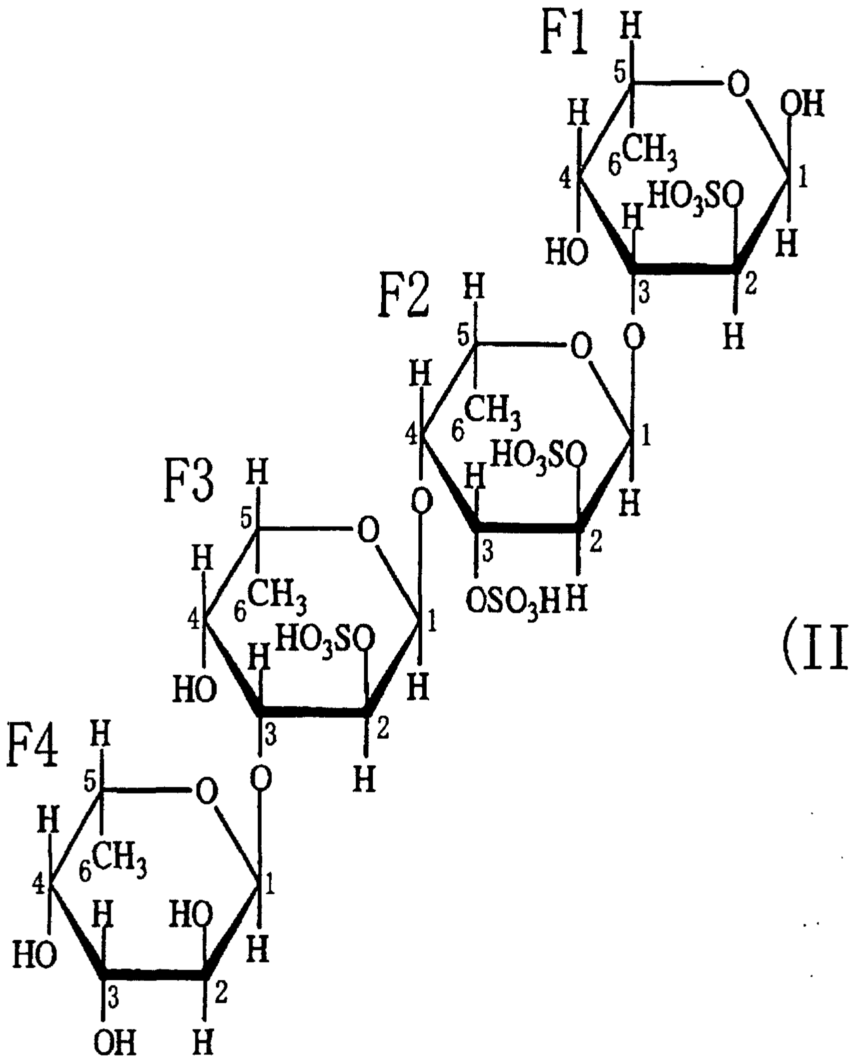

(III)

(c) Physical properties of the sulfated fucan oligosaccharide 1-(3)

The results for mass spectrometric analysis and identification in NMR analysis are shown below. The $^1$H-NMR spectrum, $^{13}$C-NMR spectrum and mass spectrum are illustrated in Figures 9, 10 and 11, respectively. In Figures 9 and 10, the longitudinal axes represent the signal intensity and the horizontal axes represent the chemical shift value (ppm). In Figure 11, the longitudinal axis represents the relative intensity (%) and the horizontal axis represents the m/z value.
Molecular weight: 1454
MS m/z; 379.1, $[M+3Na^+-7H^+]^{4-}$; 513.2, $[M+4Na^+-7H^+]^{3-}$; 781.1, $[M+5Na^+-7H^+]^{2-}$

Results of $^1$H-NMR and $^{13}$C-NMR analyses are shown in Tables 5 and 6.

Table 5

| | Chemical shift value (ppm) | |
|---|---|---|
| | $^1$H-NMR Chemical shift value, multiplicity, coupling constant | $^{13}$C-NMR |
| F1-1 | 5.43, d, 3.5 | 91.4 |
| F1-2 | 4.47, m | 74.25 or 74.3 or 74.4 |
| F1-3 | 4.01, dd, 3.5, 10.0 | 74.25 or 74.3 or 74.4 |
| F1-4 | 4.04, m | 69.9 |

Table 5 (continued)

| | Chemical shift value (ppm) | |
|---|---|---|
| | $^{1}$H-NMR Chemical shift value, multiplicity, coupling constant | $^{13}$C-NMR |
| F1-5 | 4.17, m | 66.8 |
| F1-6 | 1.18, d, 6.5 | 16.3 |
| F2-1 | 5.33, m | 95.67 or 95.71 |
| F2-2 | 4.58, dd, 3.5, 11.0 | 73.3 or 73.4 |
| F2-3 | 4.70, dd, 2.5, 11.0 | 74.8 or 74.9 |
| F2-4 | 4.22, m | 80.5 |
| F2-5 | 4.49, m | 68.7 |
| F2-6 | 1.34, d, 6.5 | 16.5 |
| F3-1 | 5.24, d, 3.5 | 99.7 |
| F3-2 | 4.52, m | 74.25 or 74.3 or 74.4 |
| F3-3 | 4.16, dd, 3.5, 10.0 | 74.25 or 74.3 or 74.4 |
| F3-4 | 4.06, d, 3.0 | 70.3 |
| F3-5 | 4.35, m | 67.9 |
| F3-6 | 1.24, d, 6.5 | 16.19 |

Table 6

| | Chemical shift value (ppm) | |
|---|---|---|
| | $^{1}$H-NMR Chemical shift value, multiplicity, coupling constant | $^{13}$C-NMR |
| F4-1 | 5.33, m | 95.67 or 95.71 |
| F4-2 | 4.59, dd, 3.5, 11.0 | 73.3 or 73.4 |
| F4-3 | 4.73, dd, 2.5, 11.0 | 74.8 or 74.9 |
| F4-4 | 4.22, m | 80.3 |
| F4-5 | 4.56, m | 68.7 |
| F4-6 | 1.33, d, 6.5 | 16.5 |
| F5-1 | 5.24, d, 3.5 | 99.5 |
| F5-2 | 4.49, m | 74.25 or 74.3 or 74.4 |
| F5-3 | 4.16, dd, 3.5, 10.0 | 72.3 |
| F5-4 | 4.04, m | 69.9 |
| F5-5 | 4.38, m | 68.0 |
| F5-6 | 1.23, d, 6.5 | 16.19 |
| F6-1 | 5.01, d, 4.0 | 96.2 |
| F6-2 | 3.72, dd, 4.0, 10.5 | 69.0 |
| F6-3 | 3.91, dd, 3.5, 10.5 | 70.3 |
| F6-4 | 3.77, d, 3.5 | 72.9 |
| F6-5 | 4.41, m | 67.8 |
| F6-6 | 1.16, d, 6.5 | 16.16 |

Table 6 (continued)

| | Chemical shift value (ppm) | |
|---|---|---|
| | $^1$H-NMR Chemical shift value, multiplicity, coupling constant | $^{13}$C-NMR |
| Saccharide composition: 6 molecules of L-fucose<br>Sulfate group: 7 molecules | | |

The numbers for peak identification in $^1$H-NMR and $^{13}$C-NMR are as indicated in formula (IV) below:

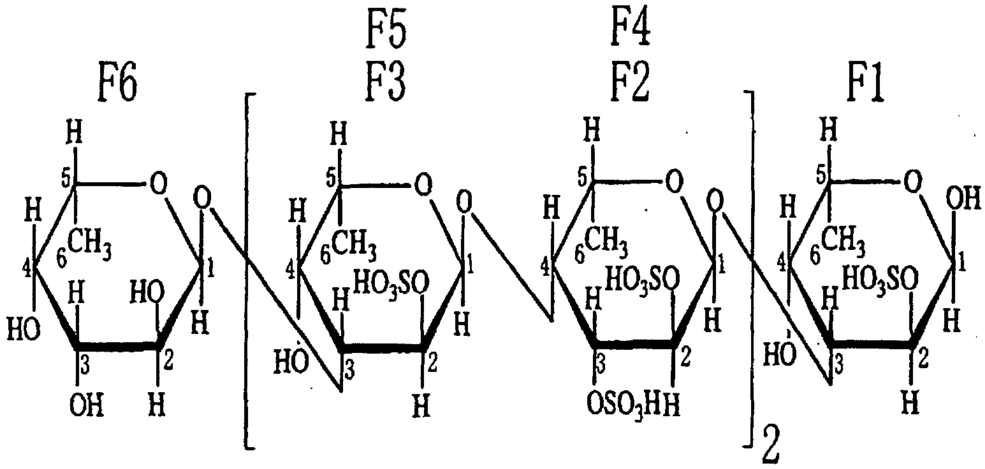

(IV)

(d) Physical properties of the sulfated fucan oligosaccharide 1-(4)

The results for mass spectrometric analysis and identification in NMR analysis are shown below. The 1H-NMR spectrum, $^{13}$C-NMR spectrum and mass spectrum are illustrated in Figures 12, 13 and 14, respectively. In Figures 12 and 13, the longitudinal axes represent the signal intensity and the horizontal axes represent the chemical shift value (ppm). In Figure 14, the longitudinal axis represents the relative intensity (%) and the horizontal axis represents the m/z value.

Molecular weight: 1986

MS m/z; 418.3, $[M+5Na^+-10H^+]^{5-}$; 528.5, $[M+6Na^+-10H^+]^{4-}$; 712.4, $[M+7Na^+-10H^+]^{3-}$; 1080.6, $[M+8Na^+-10H^+]^{2-}$

Results of $^1$H-NMR and $^{13}$C-NMR analyses are shown in Tables 7 to 9.

Table 7

| | Chemical shift value (ppm) | |
|---|---|---|
| | $^1$H-NMR Chemical shift value, multiplicity, coupling constant | $^{13}$C-NMR |
| F1-1 | 5.43, d, 3.5 | 91.4 |
| F1-2 | 4.47, dd, 4.0, 10.0 | 74.2 or 74.3 or 74.4 |
| F1-3 | 4.01, dd, 3.5, 10.5 | 74.2 or 74.3 or 74.4 |
| F1-4 | 4.04, d, 3.5 | 69.8 or 69.9 |
| F1-5 | 4.18, m | 66.8 |
| F1-6 | 1.17, d, 7.0 | 16.3 |
| F2-1 | 5.33, m | 95.5 or 95.6 or 95.7 |
| F2-2 | 4.58, dd, 3.5, 10.5 | 73.3 or 73.4 |
| F2-3 | 4.70, dd, 2.5, 11.0 | 74.8 or 74.9 |
| F2-4 | 4.23, m | 80.6 |

Table 7 (continued)

| | Chemical shift value (ppm) | |
|---|---|---|
| | $^1$H-NMR Chemical shift value, multiplicity, coupling constant | $^{13}$C-NMR |
| F2-5 | 4.49, m | 68.7 |
| F2-6 | 1.33, m | 16.5 |
| F3-1 | 5.24, m | 99.5 or 99.6 or 99.7 |
| F3-2 | 4.52, m | 74.2 or 74.3 or 74.4 |
| F3-3 | 4.16, dd, 3.0, 10.5 | 74.2 or 74.3 or 74.4 |
| F3-4 | 4.06, m | 70.2 or 70.27 or 70.32 |
| F3-5 | 4.33, m | 67.8 |
| F3-6 | 1.23, m | 16.2 |

Table 8

| | Chemical shift value (ppm) | |
|---|---|---|
| | $^1$H-NMR Chemical shift value, multiplicity, coupling constant | $^{13}$C-NMR |
| F4-1 | 5.33, m | 95.5 or 95.6 or 95.7 |
| F4-2 | 4.58, dd, 3.5, 10.5 | 73.3 or 73.4 |
| F4-3 | 4.73, dd, 2.5, 11.0 | 74.8 or 74.9 |
| F4-4 | 4.23, m | 80.2 or 80.3 |
| F4-5 | 4.56, m | 68.7 |
| F4-6 | 1.33, m | 16.5 |
| F5-1 | 5.24, m | 99.5 or 99.6 or 99.7 |
| F5-2 | 4.52, m | 74.2 or 74.3 or 74.4 |
| F5-3 | 4.16, dd, 3.0, 10.5 | 74.2 or 74.3 or 74.4 |
| F5-4 | 4.06, m | 70.2 or 70.27 or 70.32 |
| F5-5 | 4.38, m | 67.8 |
| F5-6 | 1.23, m | 16.2 |
| F6-1 | 5.33, m | 95.5 or 95.6 or 95.7 |
| F6-2 | 4.58, dd, 3.5, 10.5 | 73.3 or 73.4 |
| F6-3 | 4.73, dd, 2.5, 11.0 | 74.8 or 74.9 |
| F6-4 | 4.23, m | 80.2 or 80.3 |
| F6-5 | 4.56, m | 68.7 |
| F6-6 | 1.33, m | 16.5 |

Table 9

| | Chemical shift value (ppm) | |
|---|---|---|
| | $^1$H-NMR Chemical shift value, multiplicity, coupling constant | $^{13}$C-NMR |
| F7-1 | 5.24, m | 99.5 or 99.6 or 99.7 |
| F7-2 | 4.49, m | 74.2 or 74.3 or 74.4 |
| F7-3 | 4.16, dd, 3.0, 10.5 | 72.3 |

Table 9 (continued)

| | Chemical shift value (ppm) | |
|---|---|---|
| | $^1$H-NMR Chemical shift value, multiplicity, coupling constant | $^{13}$C-NMR |
| F7-4 | 4.04, d, 3.5 | 69.8 or 69.9 |
| F7-5 | 4.38, m | 68.0 |
| F7-6 | 1.23, m | 16.2 |
| F8-1 | 5.01, d, 3.5 | 96.2 |
| F8-2 | 3.72, dd, 4.0, 10.0 | 69.0 |
| F8-3 | 3.91, dd, 3.5, 10.5 | 70.2 or 70.27 or 70.32 |
| F8-4 | 3.77, d, 4.0 | 72.9 |
| F8-5 | 4.40, m | 67.8 |
| F8-6 | 1.16, d, 7.0 | 16.2 |
| Saccharide composition: 8 molecules of L-fucose<br>Sulfate group: 10 molecules | | |

The numbers for peak identification in $^1$H-NMR and $^{13}$C-NMR are as indicated in formula (V) below:

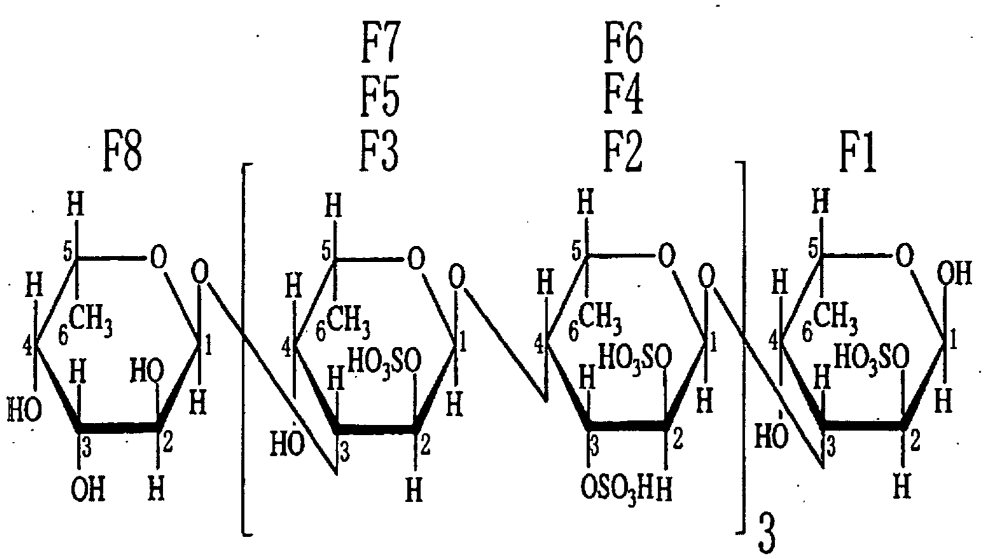

(V)

(e) Physical properties of the sulfated fucan oligosaccharide 1-(5)

The results for mass spectrometric analysis and identification in NMR analysis are shown below. The $^1$H-NMR spectrum, $^{13}$C-NMR spectrum and mass spectrum are illustrated in Figures 15, 16 and 17, respectively. In Figures 15 and 16, the longitudinal axes represent the signal intensity and the horizontal axes represent the chemical shift value (ppm). In Figure 17, the longitudinal axis represents the relative intensity (%) and the horizontal axis represents the m/z value.

Molecular weight: 2518

MS m/z; 327.6, $[M+5Na^+-13H^+]^{8-}$; 377.8, $[M+6Na^+-13H^+]^{7-}$; 444.5, $[M+7Na^+-13H^+]^{6-}$; 538.0, $[M+8Na^+-13H^+]^{5-}$; 678.1, $[M^+9Na^+-13H^+]^{4-}$; 912.4, $[M+10Na^+-13^+]^{3-}$

Results of $^1$H-NMR and $^{13}$C-NMR analyses are shown in Tables 10 to 13.

Table 10

| | Chemical shift value (ppm) | |
|---|---|---|
| | $^1$H-NMR Chemical shift value, multiplicity, coupling constant | $^{13}$C-NMR |
| F1-1 | 5:43, d, 4.0 | 91.4 |
| F1-2 | 4.47, dd, 4.0, 10.0 | 74.1 or 74.3 or 74.4 |
| F1-3 | 4.01, dd, 3.5, 10.5 | 74.1 or 74.3 or 74.4 |
| F1-4 | 4.04, d, 2.5 | 69.8 or 69.9 |
| F1-5 | 4.17, m | 66.8 |
| F1-6 | 1.17, d, 6.5 | 16.3 |
| F2-1 | 5.35, m | 95.3 or 95.6 or 95.7 |
| F2-2 | 4.60, m | 73.3 or 73.4 |
| F2-3 | 4.70, dd, 2.5, 11.0 | 74.8 or 75.0 |
| F2-4 | 4.22, m | 80.6 |
| F2-5 | 4.50, m | 68.6 or 68.7 or 68.75 or 68.81 |
| F2-6 | 1.33, m | 16.45 or 16.54 |
| F3-1 | 5.23, m | 99.3 or 99.5 or 99.7 |
| F3-2 | 4.52, m | 74.1 or 74.3 or 74.4 |
| F3-3 | 4.16, dd, 3.0, 10.5 | 74.1 or 74.3 or 74.4 |
| F3-4 | 4.06, m | 70.3 |
| F3-5 | 4.32, q, 7.0 | 67.8 |
| F3-6 | 1.23, m | 16.2 |

Table 11

| | Chemical shift value (ppm) | |
|---|---|---|
| | $^1$H-NMR Chemical shift value, multiplicity, coupling constant | $^{13}$C-NMR |
| F4-1 | 5.35, m | 95.3 or 95.6 or 95.7 |
| F4-2 | 4.60, m | 73.3 or 73.4 |
| F4-3 | 4.75, m | 74.8 or 75.0 |
| F4-4 | 4.22, m | 79.8 or 80.1 or 80.2 |
| F4-5 | 4.55, m | 68.6 or 68.7 or 68.75 or 68.81 |
| F4-6 | 1.33, m | 16.45 or 16.54 |
| F5-1 | 5.23, m | 99.3 or 99.5 or 99.7 |
| F5-2 | 4.52, m | 74.1 or 74.3 or 74.4 |
| F5-3 | 4.16, dd, 3.0, 10.5 | 74.1 or 74.3 or 74.4 |
| F5-4 | 4.06, m | 70.3 |
| F5-5 | 4.39, m | 67.8 |
| F5-6 | 1.23, m | 16.2 |
| F6-1 | 5.35, m | 95.3 or 95.6 or 95.7 |
| F6-2 | 4.60, m | 73.3 or 73.4 |

Table 11 (continued)

| | Chemical shift value (ppm) | |
|---|---|---|
| | $^1$H-NMR Chemical shift value, multiplicity, coupling constant | $^{13}$C-NMR |
| F6-3 | 4.75, m | 74.8 or 75.0 |
| F6-4 | 4.22, m | 79.8 or 80.1 or 80.2 |
| F6-5 | 4.55, m | 68.6 or 68.7 or 68.75 or 68.81 |
| F6-6 | 1.33, m | 16.45 or 16.54 |

Table 12

| | Chemical shift value (ppm) | |
|---|---|---|
| | $^1$H-NMR Chemical shift value, multiplicity, coupling constant | $^{13}$C-NMR |
| F7-1 | 5.23, m | 99.3 or 99.5 or 99.7 |
| F7-2 | 4.52, m | 74.1 or 74.3 or 74.4 |
| F7-3 | 4.16, dd, 3.0, 10.5 | 74.1 or 74.3 or 74.4 |
| F7-4 | 4.06, m | 70.3 |
| F7-5 | 4.39, m | 67.8 |
| F7-6 | 1.23, m | 16.2 |
| F8-1 | 5.35, m | 95.3 or 95.6 or 95.7 |
| F8-2 | 4.60, m | 73.3 or 73.4 |
| F8-3 | 4.75, m | 74.8 or 75.0 |
| F8-4 | 4.22, m | 79.8 or 80.1 or 80.2 |
| F8-5 | | 68.6 or 68.7 or 68.75 or 68.81 |
| F8-6 | 1.33, m | 16.45 or 16.54 |
| F9-1 | 5.23, m | 99.3 or 99.6 or 99.7 |
| F9-2 | 4.49, m | 74.1 or 74.3 or 74.4 |
| F9-3 | 4.16, dd, 3.0, 10.5 | 72.2 |
| F9-4 | 4.04, d, 2.5 | 69.8 or 69.9 |
| F9-5 | 4.39, m | 68.0 |
| F9-6 | 1.23, m | 16.2 |

Table 13

| | Chemical shift value (ppm) | |
|---|---|---|
| | $^1$H-NMR Chemical shift value, multiplicity, coupling constant | $^{13}$C-NMR |
| F10-1 | 5.01, d, 4.0 | 96.1 |
| F10-2 | 3.72, dd, 4.0, 10.0 | 69.0 |
| F10-3 | 3.91, dd, 3.5, 10.5 | 70.3 |
| F10-4 | 3.77, d, 3.0 | 72.9 |
| F10-5 | 4.40, m | 67.8 |
| F10-6 | 1.16, d, 7.0 | 16.2 |

Table 13 (continued)

| | Chemical shift value (ppm) | |
|---|---|---|
| | $^{1}$H-NMR Chemical shift value, multiplicity, coupling constant | $^{13}$C-NMR |
| Saccharide composition: 10 molecules of L-fucose<br>Sulfate group: 13 molecules | | |

The numbers for peak identification in $^{1}$H-NMR and $^{13}$C-NMR are as indicated in formula (VI) below:

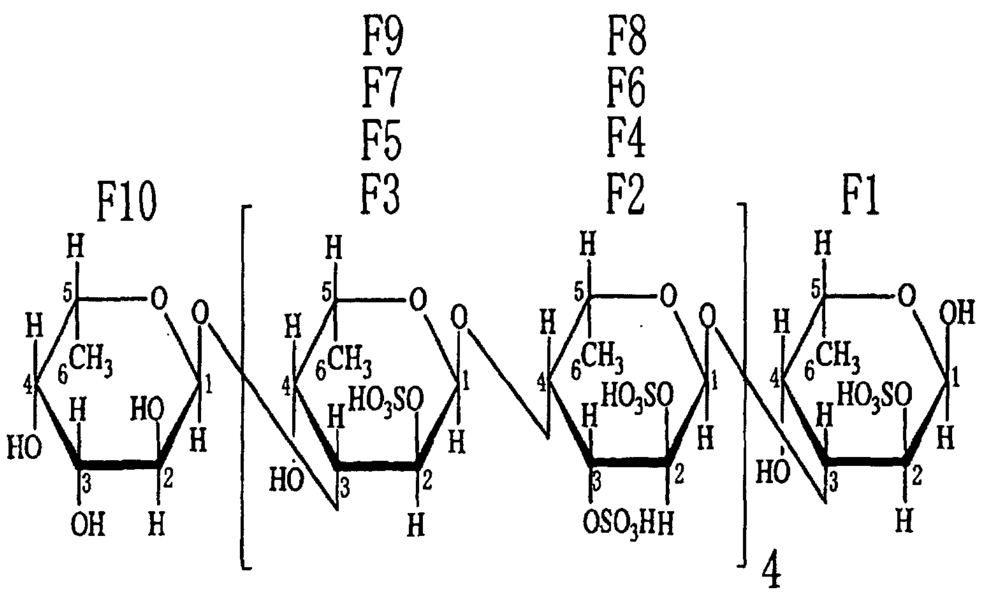

(VI)

(5) Enzyme species included in the sulfated fucan-degrading enzyme of the present invention

All of the oligosaccharides 1-(1) to (5) as described above in Example 2 can be represented by general formula (I). Thus, it is considered that they were generated from polysaccharides having the same backbone. The fact that the saccharides at the reducing ends for all of the oligosaccharides are L-fucose revealed that an endo-type α-(1-4)L-fucosidase existed in the sulfated fucan-degrading enzyme. The fact that the oligosaccharides have structures in which disaccharides are repeated revealed that the endo-type α-(1-4)L-fucosidase is an enzyme that cleaves α-(1-4)L-fucosyl bonds. Furthermore, the fact that sulfuric acid, L-fucose and acetic acid were detected in the reaction mixtures for obtaining the oligosaccharides suggested that the sulfated fucan-degrading enzyme preparation obtained in Example 1 contained a sulfatase activity, a fucosidase activity and a deacetylase activity.

(6) Measurement of endo-type α-(1-4)L-fucosidase activity

The sulfated fucan oligosaccharide 1-(4) as described in Example 2 was used to establish a system for measuring an endo-type α-(1-4)L-fucosidase activity. First, the sulfated fucan oligosaccharide 1-(4) was fluorescently labeled at the reducing end with 2-aminopyridine according to the method as described in Example 2-(4). The endo-type α-(1-4) L-fucosidase activity was measured as follows.

Briefly, 75 μl of 100 mM sodium phosphate buffer (pH 7.0), 7.5 μl of a bovine serum albumin solution (2 mg/ml), 3.75 μl of 4 M sodium chloride, 50.25 μl of water, 6 μl of the sulfated fucan oligosaccharide 1-(4) fluorescently labeled at the reducing end with 2-aminopyridine (10 pmol/μl) and 7.5 μl of an endo-type α-(1-4)L-fucosidase solution were mixed together. After reacting at 30°C for 3 hours, the reaction mixture was treated at 100°C for 10 minutes and analyzed using HPLC. As controls, a reaction mixture obtained by a reaction in which the solvent for the endo-type α-(1-4)L-fucosidase was used in place of the enzyme solution and a reaction mixture obtained by a reaction in which water was used in place of the substrate were similarly analyzed. The HPLC was carried out as follows:

Column: TSK-gel Octyl-80Ts (4.6 x 250 mm, Tosoh);
Column temperature: 40°C;

Eluents: mixtures of 200 mM triethylamine acetate buffer (pH 5.5) and acetonitrile at the following ratios:

Solution A: 95:5;
Solution B: 70:30;
Solution C: 40:60;

Compositions of eluents upon analysis using HPLC

0-7 min. Solution A;
7-25 min. gradient from Solution A to a mixture of Solutions A and B (A:B=10:90);
25-26 min. gradient from the mixture of Solutions A and B (A:B=10:90) to Solutions C;
26-34 min. Solution C;

Flow rate: 1 ml/minute;
Detection: fluorescence (excitation wavelength: 320 nm; emission wavelength: 400 nm)

One unit of an endo-type α-(1-4)L-fucosidase activity is defined as an amount of an enzyme that cleaves 1 μmole of fucosyl bond in 1 minute in the above-mentioned reaction system. The amount of cleaved fucosyl bond was calculated according to the following equation:

$$S / (180 \times 0.0075 \times 10^{6}) = U/ml$$

S: amount of cleaved substrate in moles (pmole);
180: the reaction time (minutes); and
0.0075: the volume of the enzyme solution (ml).

The amount of protein was determined by measuring the absorbance of the enzyme solution at 280 nm. The calculation was carried out assuming the absorbance of a solution containing a protein at a concentration of 1 mg/ml as 1.0.

Example 3: Endo-type α-(1-4)L-fucosidase

(1) Partial purification of endo-type α-(1-4)L-fucosidase

*Fucophilus fucoidanolyticus* SI-1234 was cultured as described in Example 1 and cells were collected by centrifugation from 60 L of the culture.

The cells were suspended in 2360 ml of 10 mM imidazole-hydrochloride buffer (pH 7.0) containing 10 mM calcium chloride, 400 mM sodium chloride and 5 mM sodium azide, sonicated and centrifuged to obtain a supernatant. The supernatant was adequately dialyzed against 10 mM imidazole-hydrochloride buffer (pH 7.0) containing 50 mM sodium chloride and 5 mM sodium azide. The formed precipitate was removed by centrifugation to obtain a supernatant as a crude enzyme solution of the endo-type α-(1-4)L-fucosidase.

The crude enzyme solution of the endo-type α-(1-4)L-fucosidase was loaded onto a 3000-ml DEAE-Cellulofine A-800 column equilibrated with the same buffer. After adding the crude enzyme solution of the endo-type α-(1-4)L-fucosidase and adequately washing with the same buffer, elution was carried out with a gradient of 50 to 600 mM sodium chloride to collect fractions each containing 60 ml of the eluate.

The endo-type α-(1-4)L-fucosidase activities were measured for the respective fractions. The active fraction was loaded onto a 150-ml hydroxyapatite column equilibrated with 10 mM imidazole-hydrochloride buffer (pH 7.0) containing 200 mM sodium chloride and 20 mM potassium dihydrogenphosphate. After adding the enzyme solution and adequately washing with the equilibration buffer, elution was carried out with a gradient of 20 to 400 mM potassium dihydrogenphosphate to collect fractions each containing 10 ml of the eluate.

The endo-type α-(1-4)L-fucosidase activities were measured for the respective fractions. The active fraction was concentrated using an ultrafiltration device equipped with an ultrafiltration membrane with exclusion molecular weight of 10,000, and loaded onto a Sephacryl S-200 column (4.4 x 100 cm) equilibrated with 10 mM imidazole-hydrochloride buffer (pH 7.5) containing 100 mM sodium chloride and 5 mM sodium azide. After adding the enzyme solution, elution was carried out using the same buffer to collect fractions each containing 13 ml of the eluate. The purification procedure is summarized in Table 14.

Table 14

| Step | Protein (mg) | Total activity (mU) | Specific activity (mU/mg) | Yield (%) |
|---|---|---|---|---|
| Extract | 29,430 | 3,070 | 0.104 | 100 |
| Dialysis | 21,900 | 3,330 | 0.152 | 108 |
| DEAE-Cellulofine | 478 | 685 | 1.43 | 22.3 |
| Hydroxyapatite | 20.7 | 219 | 10.6 | 7.13 |
| Sephacryl S-200 | 2.66 | 32.7 | 12.3 | 1.07 |

The following experiments were carried out in order to confirm that the endo-type α-(1-4)L-fucosidase cleaves α-(1-4)L-fucosyl bonds in an endo-type manner.

Specifically, the endo-type α-(1-4)L-fucosidase was allowed to act on the sulfated fucan oligosaccharide 1-(4) fluorescently labeled at the reducing end with 2-aminopyridine. An aliquot of the reaction mixture was dried and oligosaccharides in the dried aliquot were fluorescently labeled with 2-aminopyridine and analyzed using HPLC. The sulfated fucan oligosaccharide 1-(2) fluorescently labeled with 2-aminopyridine was used as a standard. As a result, it was shown that all the oligosaccharides generated by cleavage in the enzymatic reaction were the same substance as the sulfated fucan oligosaccharide 1-(2). Thus, it was confirmed that the endo-type α-(1-4)L-fucosidase is an enzyme that cleaves α-(1-4)L-fucosyl bonds in a sulfated fucan derived from *Fucus vesiculosus* in an endo-type manner. The optimal pH and the optimal temperature of the endo-type α-(1-4)L-fucosidase are shown in Figures 18 and 19.

However, when the endo-type α-(1-4)L-fucosidase alone was allowed to act on a sulfated fucan fraction from *Fucus vesiculosus*, the enzyme could not convert the sulfated fucan fraction into smaller molecules. As described above, it was known that L-fucose, sulfuric acid and acetic acid were generated in the reaction mixture upon preparation of a sulfated fucan oligosaccharide using the sulfated fucan-degrading enzyme. Based on the fact, it was considered that the presence of a fucosidase, a sulfatase, a deacetylase or the like is necessary for degradation of a sulfated fucan using the endo-type α-(1-4)L-fucosidase.

Then, an activity of which the coexistence with the endo-type α-(1-4)L-fucosidase results in conversion of a sulfated fucan from *Fucus vesiculosus* into smaller molecules was searched.

(2) Enzyme A that converts a sulfated fucan into smaller molecules

In order to prepare an enzyme of which the coexistence with the endo-type α-(1-4)L-fucosidase results in conversion of a sulfated fucan from *Fucus vesiculosus* into smaller molecules, a system for measuring the activity of the enzyme was established as follows.

Briefly, 12 μl of 500 mM imidazole-hydrochloride buffer (pH 7.5), 4.8 μl of 2.5% solution of the sulfated fucan fraction from *Fucus vesiculosus*, 6 μl of 1 M calcium chloride, 9 μl of 4 M sodium chloride, 40.2 μl of water, 24 μl of the endo-type α-(1-4)L-fucosidase (0.35-0.4 mU/ml) and 24 μl of the enzyme A that converts a sulfated fucan into smaller molecules were mixed together. After reacting at 30°C for 3 hours, the reaction mixture was treated at 100°C for 1.0 minutes and analyzed using HPLC. As a control, a reaction mixture obtained by a reaction in which the solvent for the enzyme A that converts a sulfated fucan into smaller molecules was used in place of the enzyme solution was similarly analyzed. The HPLC was carried out as follows:

Column: SB-806HQ (4.6 x 250 mm, Showa Denko);
Column temperature: 25°C;
Eluent: 50 mM sodium chloride containing 5 mM sodium azide;
Flow rate: 1 ml/minute;
Detection: differential refractive index detector

One unit of an enzymatic activity of the enzyme A that converts a sulfated fucan into smaller molecules is defined as an amount of an enzyme that increases cleavage of 1 μmol of fucosyl bond by the endo-type α-(1-4)L-fucosidase in 1 minute in the above-mentioned reaction system. The amount of cleaved fucosyl bond was calculated according to the following equation:

$$(120/SMr) \times (SMr/PMr-1) \times 1/180 \times 0.024 = U/ml$$

120: the amount of the sulfated fucan used as a substrate (μg);
SMr: the average molecular weight of the substrate;
PMr: the average molecular weight of the reaction product;
180: the reaction time (minutes); and
0.024: the volume of the enzyme solution (ml).

The amount of protein was determined by measuring the absorbance of the enzyme solution at 280 nm. The calculation was carried out assuming the absorbance of a solution containing a protein at a concentration of 1 mg/ml as 1.0.

Example 4: Enzyme A that converts a sulfated fucan into smaller molecules

(1) Partial purification of enzyme A that converts a sulfated fucan into smaller molecules

*Fucophilus fucoidanolyticus* SI-1234 was cultured as described in Example 1 and cells were collected by centrifugation from 60 L of the culture.

The cells were suspended in 2780 ml of 10 mM imidazole-hydrochloride buffer (pH 7.0) containing 10 mM calcium chloride, 400 mM sodium chloride, 5 mM sodium azide and 5 mM β-mercaptoethanol, sonicated and centrifuged to obtain a supernatant. The supernatant was adequately dialyzed against 10 mM imidazole-hydrochloride buffer (pH 7.0) containing 10 mM calcium chloride, 10 mM sodium chloride, 5 mM β-mercaptoethanol and 5 mM sodium azide. The formed precipitate was removed by centrifugation to obtain a supernatant.

The supernatant was loaded onto a 3000-ml DEAE-Cellulofine A-800 column equilibrated with the same buffer. After adding the supernatant and adequately washing with the same buffer, elution was carried out with a gradient of 10 to 600 mM sodium chloride to collect fractions each containing 60 ml of the eluate.

Activities of the enzyme A that converts a sulfated fucan into smaller molecules were measured for the respective fractions. The active fraction (fraction eluted with 60-110 mM sodium chloride) was loaded onto a 200-ml DEAE-Cellulofine A-800 column equilibrated with 10 mM imidazole-hydrochloride buffer (pH 7.0) containing 10 mM calcium chloride, 80 mM sodium chloride and 5 mM β-mercaptoethanol. After adding the enzyme solution and adequately washing with the equilibration buffer, elution was carried out with a gradient of 80 to 240 mM sodium chloride to collect fractions each containing 20 ml of the eluate.

Activities of the enzyme A that converts a sulfated fucan into smaller molecules were measured for the respective fractions. The active fraction was loaded onto a 20-ml Sulfate-Cellulofine column equilibrated with 10 mM imidazole-hydrochloride buffer (pH 7.0) containing 200 mM sodium chloride, 10 mM calcium chloride and 5 mM β-mercaptoethanol. After adding the enzyme solution and adequately washing with the same buffer, elution was carried out with a gradient of 200 to 700 mM sodium chloride to collect fractions each containing 2 ml of the eluate. The purification procedure is summarized in Table 15.

Table 15

| Step | Protein (mg) | Total activity (mU) | Specific activity (mU/mg) | Yield (%) |
|---|---|---|---|---|
| Extract | 33,600 | 731 | 0.0218 | 100 |
| Dialysis | 30,900 | 736 | 0.0238 | 101 |
| DEAE-Cellulofine | 567 | 97.2 | 0.171 | 13.3 |
| DEAE Cellulofine | 102 | 43.6 | 4.27 | 5.96 |
| Sulfate-Cellulofine | 9.66 | 8.40 | 0.870 | 1.14 |

The following experiments were carried out in order to examine the mode of action of the enzyme A that converts a sulfated fucan into smaller molecules.

Specifically, the enzyme A that converts a sulfated fucan into smaller molecules was allowed to act on the sulfated fucan from *Fucus vesiculosus*. The amount of free sulfuric acid contained in an aliquot of the reaction mixture was analyzed using HPLC. Sodium sulfate was used as a standard. As a result, it was shown that the enzymatic reaction resulted in free sulfuric acid. Thus, it was confirmed that the enzyme A that converts a sulfated fucan into smaller molecules is an enzyme that acts on the sulfated fucan from *Fucus vesiculosus* to cleave sulfate groups, and has a sulfated fucan sulfatase activity.

However, when the endo-type α-(1-4)L-fucosidase and the enzyme A that converts a sulfated fucan into smaller molecules were allowed to act on a sulfated fucan fraction from *Fucus vesiculosus*, the enzymes could not

convert the sulfated fucan fraction into smaller molecules to such an extent that the oligosaccharides as described in Example 2 were generated. As described above, it was known that L-fucose and acetic acid were also generated in the reaction mixture upon preparation of a sulfated fucan oligosaccharide using the sulfated fucan-degrading enzyme. Based on the fact, it was considered that the presence of a fucosidase, a deacetylase or the like is necessary for degradation of a sulfated fucan from *Fucus vesiculosus* in addition to the endo-type α-(1-4)L-fucosidase and the enzyme A that converts a sulfated fucan into smaller molecules.

Then, an activity of which the coexistence with the endo-type α-(1-4)L-fucosidase and the enzyme A that converts a sulfated fucan into smaller molecules promotes conversion of a sulfated fucan from *Fucus vesiculosus* into smaller molecules was searched.

(2) Exo-type L-fucosidase

In order to prepare an exo-type L-fucosidase, a system for measuring the activity of the enzyme was established as follows.

Briefly, 12 μl of 500 mM imidazole-hydrochloride buffer (pH 7.5), 4.8 μl of 2.5% solution of the sulfated fucan fraction from *Fucus vesiculosus,* 6 μl of 1 M calcium chloride, 9 μl of 4 M sodium chloride, 16.2 μl of water, 24 μl of the endo-type α-(1-4)L-fucosidase (0.35-0.4 mU/ml), 24 μl of the enzyme A that converts a sulfated fucan into smaller molecules (0.04-0.05 mU/ml) and 24 μl of the exo-type L-fucosidase were mixed together. After reacting at 30°C for 3 hours, the reaction mixture was treated at 100°C for 10 minutes and analyzed using HPLC. As a control, a reaction mixture obtained by a reaction in which the solvent for the exo-type L-fucosidase was used in place of the enzyme solution was similarly analyzed. The HPLC was carried out as follows:

Column: SB-806HQ (4.6 x 250 mm, Showa Denko);
Column temperature: 25°C;
Eluent: 50 mM sodium chloride containing 5 mM sodium azide;
Flow rate: 1 ml/minute;
Detection: differential refractive index detector

One unit of an exo-type L-fucosidase activity is defined as an amount of an enzyme that increases cleavage of 1 μmole of fucosyl bond in 1 minute in the above-mentioned reaction system. The amount of increased fucosyl bond was calculated according to the following equation:

$$(120/SMr) \times (SMr/PMr-1) \times 1/180 \times 0.024 = U/ml$$

120: the amount of the sulfated fucan used as a substrate (μg);
SMr: the average molecular weight of the substrate;
PMr: the average molecular weight of the reaction product;
180: the reaction time (minutes); and
0.024: the volume of the enzyme solution (ml).

The amount of protein was determined by measuring the absorbance of the enzyme solution at 280 nm. The calculation was carried out assuming the absorbance of a solution containing a protein at a concentration of 1 mg/ml as 1.0.

Example 5: Partial purification of exo-type L-fucosidase

*Fucophilus fucoidanolyticus* SI-1234 was cultured as described in Example 2 and cells were collected by centrifugation from 60 L of the culture.

The cells were suspended in 2780 ml of 10 mM imidazole-hydrochloride buffer (pH 7.0) containing 10 mM calcium chloride, 400 mM sodium chloride, 5 mM sodium azide and 5 mM β-mercaptoethanol, sonicated and centrifuged to obtain a supernatant. The supernatant was adequately dialyzed against 10 mM imidazole-hydrochloride buffer (pH 7.0) containing 10 mM calcium chloride, 10 mM sodium chloride, 5 mM β-mercaptoethanol and 5 mM sodium azide. The formed precipitate was removed by centrifugation to obtain a supernatant.

The supernatant was loaded onto a 3000-ml DEAE-Cellulofine A-800 column equilibrated with the same buffer. After adding the supernatant and adequately washing with the same buffer, elution was carried out with a gradient of 10 to 600 mM sodium chloride to collect fractions each containing 60 ml of the eluate.

The exo-type L-fucosidase activities were measured for the respective fractions. The active fraction (fraction

eluted with 170-205 mM sodium chloride) was adequately dialyzed against 10 mM imidazole-hydrochloride buffer (pH 7.0) containing 10 mM calcium chloride, 50 mM sodium chloride and 5 mM β-mercaptoethanol, and loaded onto a 50-ml Sulfate-Cellulofine column equilibrated with the same buffer. After adding the enzyme solution and adequately washing with the equilibration buffer, elution was carried out with a gradient of 50 mM to 1 M sodium chloride to collect fractions each containing 5 ml of the eluate.

The exo-type L-fucosidase activities were measured for the respective fractions. The active fraction was loaded onto a 20-ml Sulfate-Cellulofine column equilibrated with 10 mM imidazole-hydrochloride buffer (pH 7.5) containing 100 mM sodium chloride, 10 mM calcium chloride, 5 mM β-mercaptoethanol and 5 mM sodium azide. After adding the enzyme solution and adequately washing with the same buffer, elution was carried out with a gradient of 100 to 700 mM sodium chloride to collect fractions each containing 2.3 ml of the eluate. The purification procedure is summarized in Table 16.

Table 16

| Step | Protein (mg) | Total activity (mU) | Specific activity (mU/mg) | Yield (%) |
|---|---|---|---|---|
| Extract | 33,600 | 12,400 | 0.369 | 100 |
| Dialysis | 30,900 | 11,900 | 0.385 | 96.0 |
| DEAE-Cellulofine | 231 | 778 | 3.37 | 6.27 |
| Sulfate-Cellulofine | 42.7 | 1,180 | 27.6 | 9.52 |
| Sulfate-Cellulofine | 8.32 | 1, 090 | 131 | 8.79 |

The following experiments were carried out in order to examine the mode of action of the exo-type L-fucosidase.

Specifically, the exo-type L-fucosidase was allowed to act on the sulfated fucan from *Fucus vesiculosus*. The amount of free L-fucose contained in an aliquot of the reaction mixture was determined using a kit for quantifying free L-fucose. As a result, it was shown that the enzymatic reaction resulted in free L-fucose. Thus, it was confirmed that the exo-type L-fucosidase is an enzyme that acts on the sulfated fucan from *Fucus vesiculosus* to cleave L-fucose residues, and is an exo-type L-fucosidase.

When the endo-type α-(1-4)L-fucosidase, the enzyme A that converts a sulfated fucan into smaller molecules and the exo-type L-fucosidase were allowed to act on the sulfated fucan fraction from *Fucus vesiculosus,* the sulfated fucan was converted into smaller molecules to generate oligosaccharides having molecular weights almost equivalent to those as described in Example 2. As described above, it was known that acetic acid was also generated in the reaction mixture upon preparation of a sulfated fucan oligosaccharide using the sulfated fucan-degrading enzyme.

Then, the endo-type α-(1-4)L-fucosidase and the enzyme A that converts a sulfated fucan into smaller molecules were allowed to act on a substrate, i.e., the sulfated fucan from *Fucus vesiculosus* which had been or had not been treated with 1 N sodium hydroxide to hydrolyze O-acetyl groups. As a result, it was clearly shown that conversion into smaller molecules proceeded only for the sulfated fucan from *Fucus vesiculosus* from which O-acetyl groups had been removed. Thus, it was confirmed that the deacetylation of the sulfated fucan is necessary for enzymatic conversion of the sulfated fucan from *Fucus vesiculosus* into smaller molecules.

Based on these results, it was considered that the endo-type α-(1-4)L-fucosidase, the enzyme A that converts a sulfated fucan into smaller molecules and the exo-type L-fucosidase contained sulfated fucan deacetylase. Then, the enzymatic activity was measured using the following reaction system.

Briefly, 6 μl of 500 mM imidazole-hydrochloride buffer (pH 7.5), 7.2 μl of 2.5% solution of the sulfated fucan fraction from *Fucus vesiculosus*, 3 μl of 1 M calcium chloride, 4.5 μl of 4 M sodium chloride, 33.3 μl of water and 6 μl of a sulfated fucan deacetylase-containing enzyme solution were mixed together. After reacting at 30°C for 3 hours, the reaction mixture was treated at 100°C for 10 minutes, and the amount of acetic acid was determined using a commercially available kit for quantifying acetic acid. As a control, a reaction mixture obtained by a reaction in which the solvent for the sulfated fucan deacetylase-containing enzyme solution was used in place of the enzyme solution was similarly analyzed. As a result, it was shown that both of the partially purified preparations of the enzyme A that converts a sulfated fucan into smaller molecules and the exo-type L-fucosidase as described in Examples above contained sulfated fucan deacetylase.

Based on these results, it was shown that the endo-type α-(1-4)L-fucosidase, the enzyme A that converts a sulfated fucan into smaller molecules, the exo-type L-fucosidase and the sulfated fucan deacetylase are involved in degradation of the sulfated fucan from *Fucus vesiculosus*.

Industrial Applicability

The present invention provides a novel sulfated fucan-degrading enzyme which can be used for structural analyses of sulfated fucans and reproducible production of sulfated fucan oligosaccharides, and a method for producing the enzyme. The present invention also provides a sulfated fucan oligosaccharide which can be produced using the enzyme and which is useful as a reagent for glycotechnology, and a method for producing the same.

## Claims

1. A sulfated fucan-degrading enzyme that degrades a sulfated fucan derived from a brown alga of the order Fucales, which is obtainable from a culture of a bacterium of the genus *Fucophilus.*

2. The sulfated fucan-degrading enzyme according to claim 1, which is an endo-type α-(1-4)L-fucosidase having the following physical and chemical properties:

   (I) acting on a sulfated fucan oligosaccharide of formula (V) to cleave an α-(1-4)L-fucosyl bond in an endo-type manner:

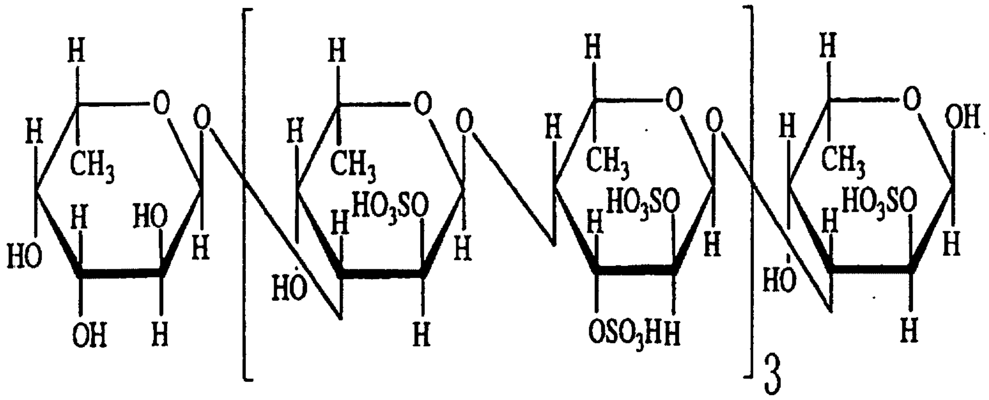

(V)

(II) having an optimal pH of about 6 to 8; and

(III) having an optimal temperature of about 10 to 40°C.

3. A method for producing the sulfated fucan-degrading enzyme defined by claim 1, the method comprising culturing a bacterium of the genus *Fucophilus,* and collecting the enzyme from the culture.

4. A sulfated fucan oligosaccharide which is obtainable by allowing the sulfated fucan-degrading enzyme defined by claim 1 to act on a sulfated polysaccharide derived from a brown alga.

5. A method for producing a sulfated fucan oligosaccharide, the method comprising allowing the sulfated fucan-degrading enzyme defined by claim 1 to act on a sulfated polysaccharide derived from a brown alga, and collecting a sulfated fucan oligosaccharide.

6. A saccharide of general formula (I) or a salt thereof:

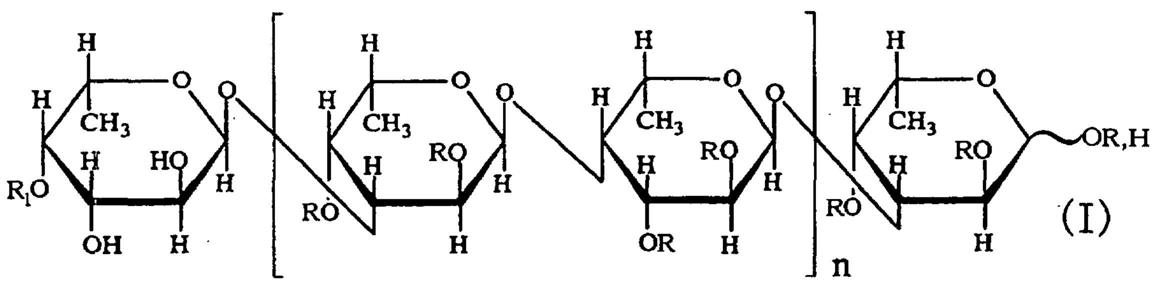

(I)

wherein R is H, $SO_3H$, $CH_3CO$ or general formula (VII); $R_1$ is H, $SO_3H$ or $CH_3CO$; at least one of Rs and $R_1$ is $SO_3H$; n is an integer of 1 or more:

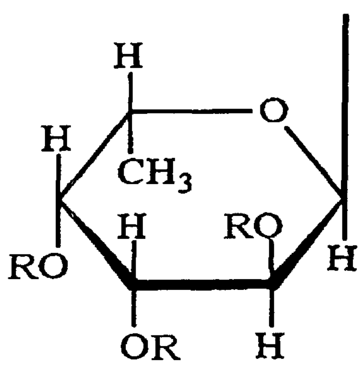

(VII)

wherein R is H, $SO_3H$ or $CH_3CO$.

**7.** A sulfated fucan oligosaccharide of general formula (I) or a salt thereof:

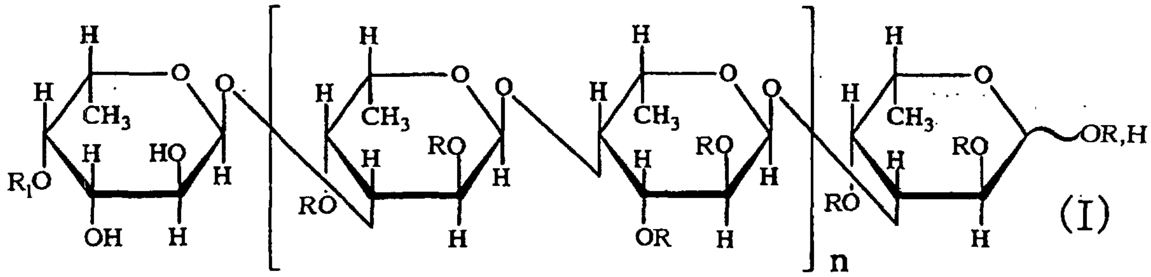

(I)

wherein R is H, $SO_3H$, $CH_3CO$ or general formula (VII); $R_1$ is H, $SO_3H$ or $CH_3CO$; at least one of Rs and $R_1$ is $SO_3$ H; n is an integer of 1 to 4:

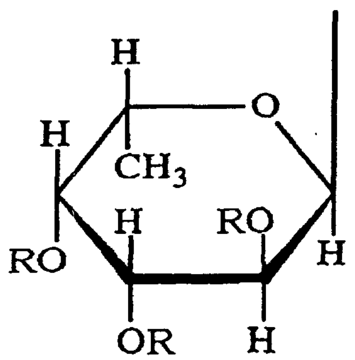

(VII)

wherein R is H, $SO_3H$ or $CH_3CO$.

**8.** A method for producing a sulfated fucan oligosaccharide of general formula (VIII), the method comprising allowing the sulfated fucan-degrading enzyme defined by claim 1 to act on a sulfated polysaccharide derived from a brown alga of the order Fucales to produce a sulfated fucan oligosaccharide of general formula (VIII):

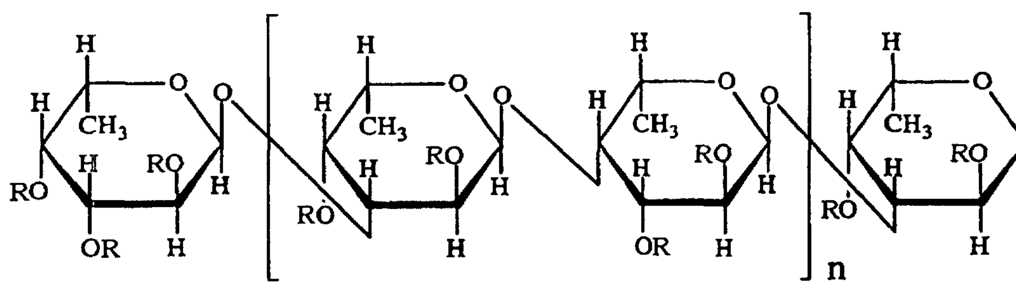

(VIII)

wherein R is H, $SO_3H$, $CH_3CO$ or general formula (VII); at least one of Rs is $SO_3H$; n is an integer of 1 or more:

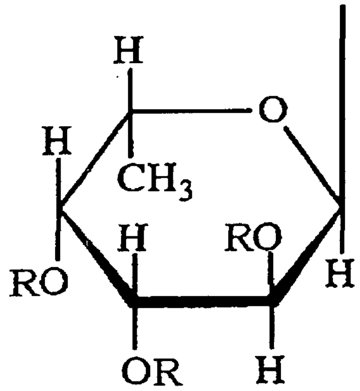

(VII)

wherein R is H, $SO_3H$ or $CH_3CO$.

**9.** The method according to claim 8, wherein the brown alga of the order Fucales is *Fucus vesiculosus* or *Ascophyllum nodosum*.

Fig. 1

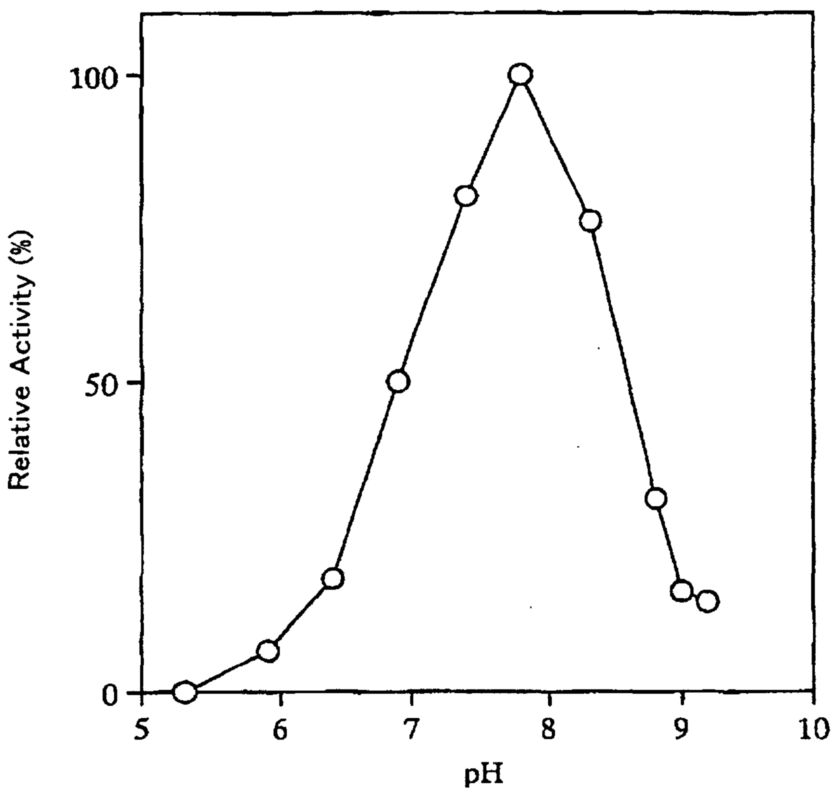

Fig. 2

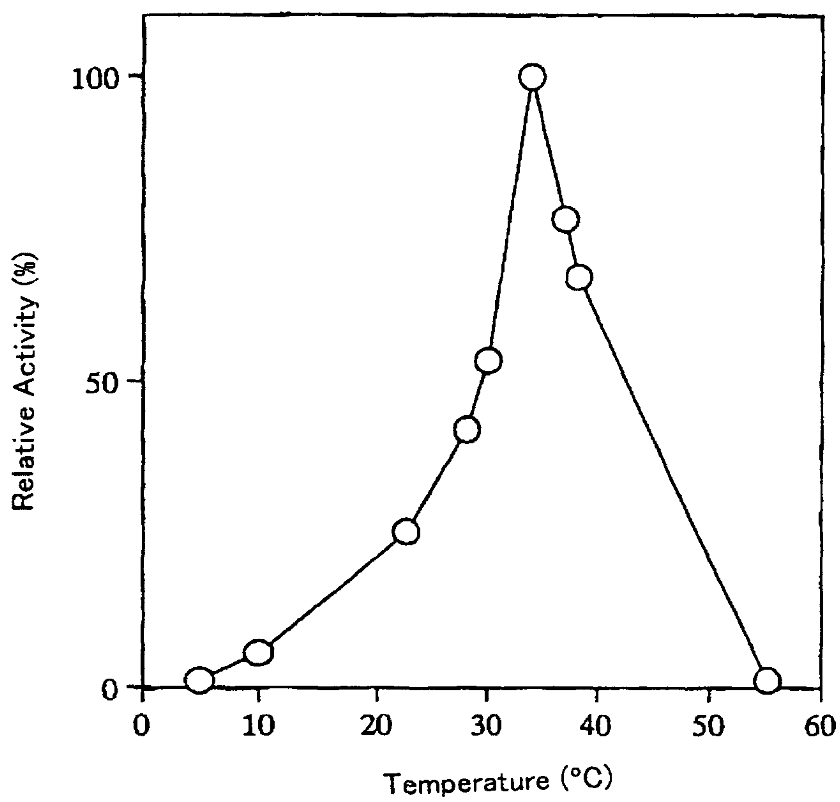

Fig. 3

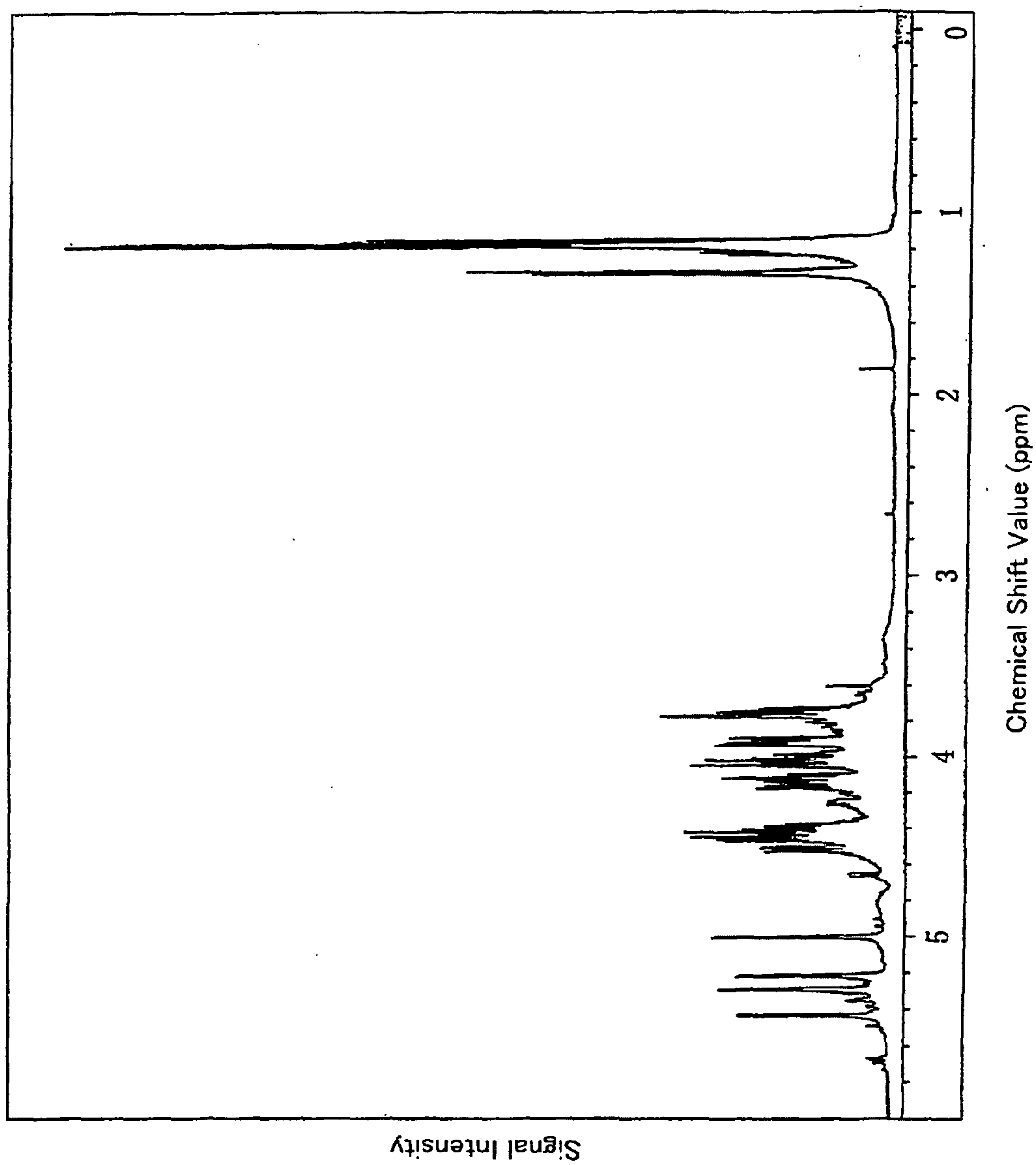

Fig. 4

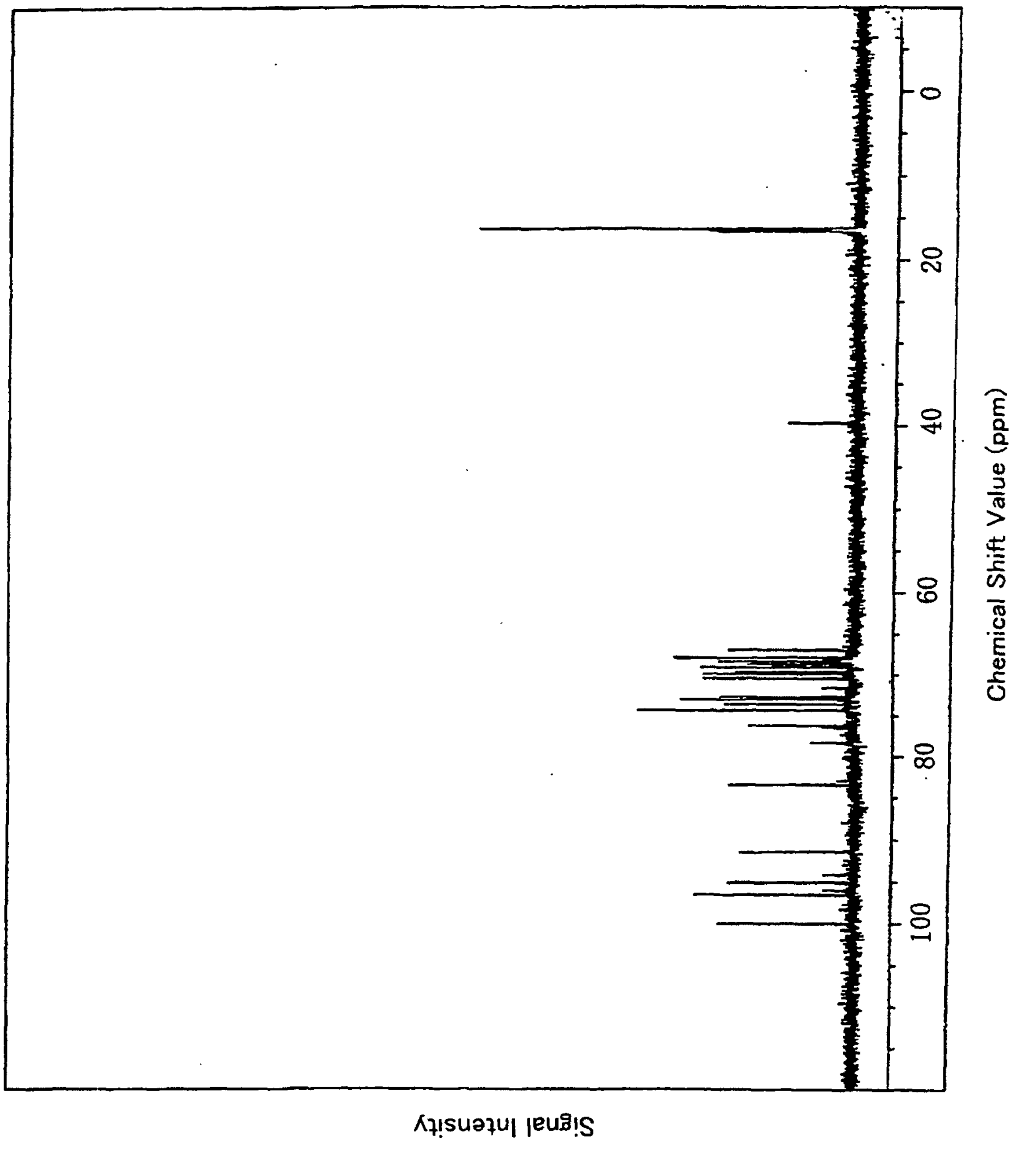

Fig. 5

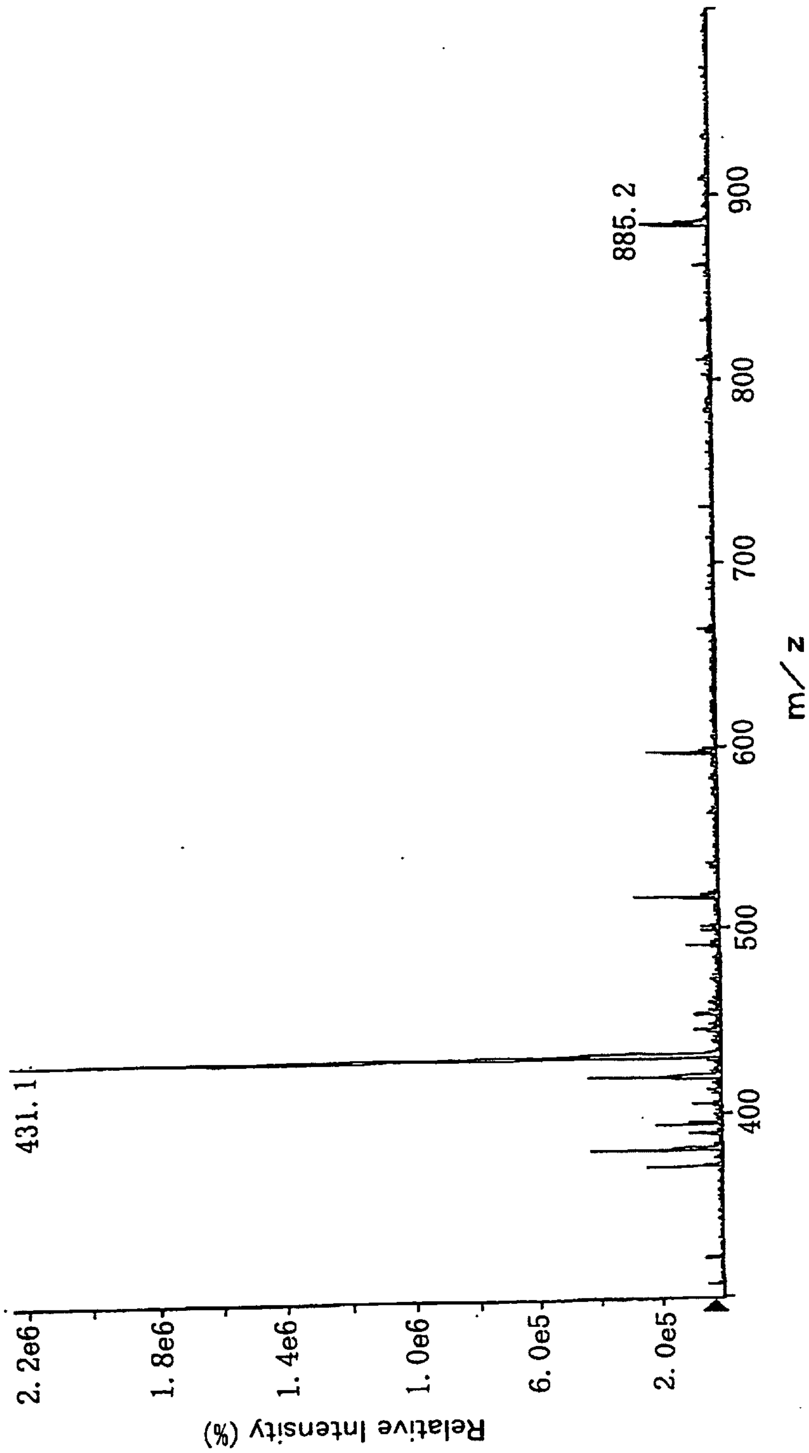
431. 1
885. 2
2. 2e6
1. 8e6
1. 4e6
1. 0e6
6. 0e5
2. 0e5
Relative Intensity (%)
400
500
600
700
800
900
m/z

Fig. 6

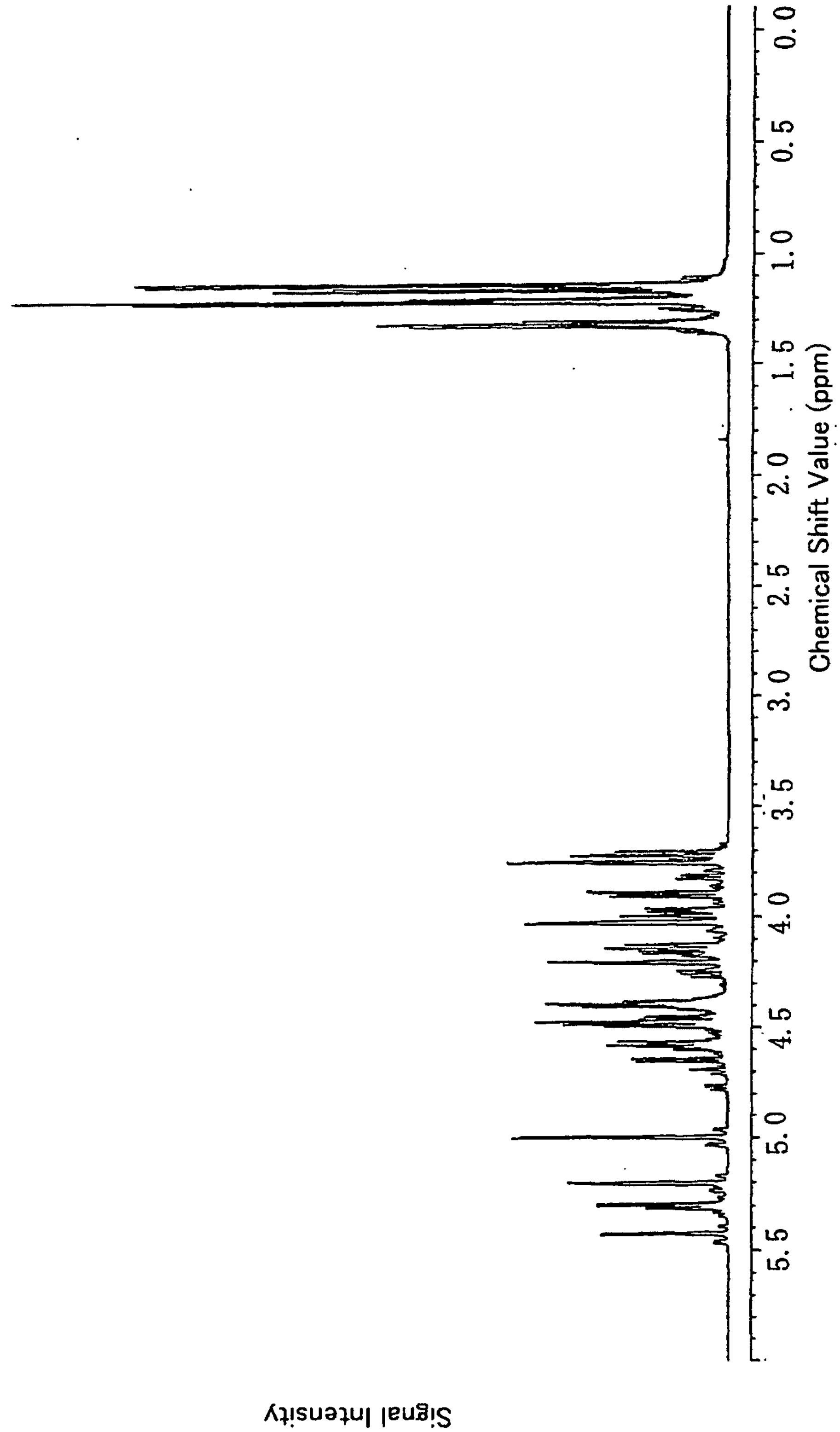
0.0
0.5
1.0
1.5
2.0
2.5
3.0
3.5
4.0
4.5
5.0
5.5
Chemical Shift Value (ppm)
Signal Intensity

Fig. 7

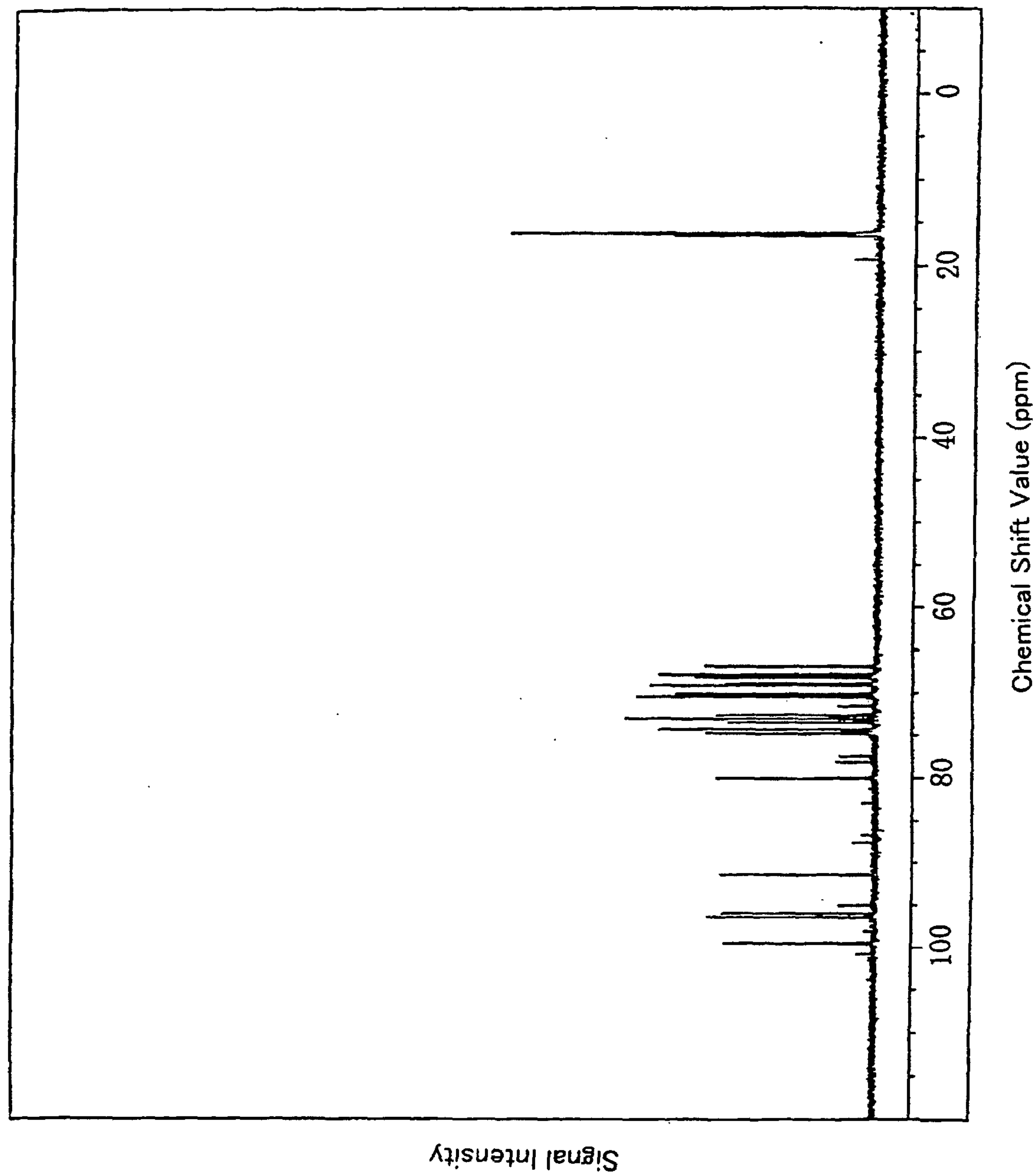

Fig. 8

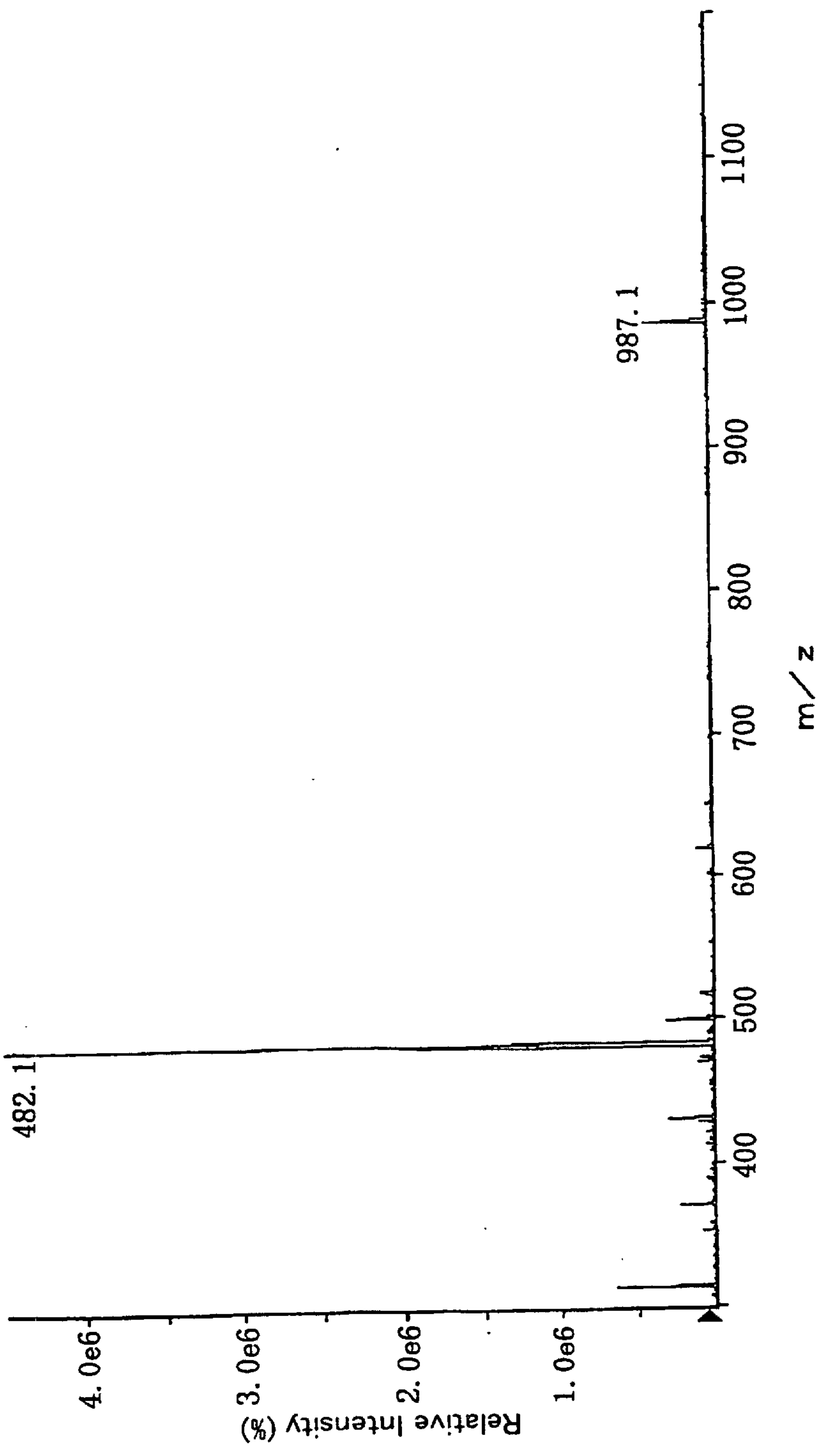
482. 1
987. 1
400
500
600
700
800
900
1000
1100
m/z
4. 0e6
3. 0e6
2. 0e6
1. 0e6
Relative Intensity (%)

Fig. 9

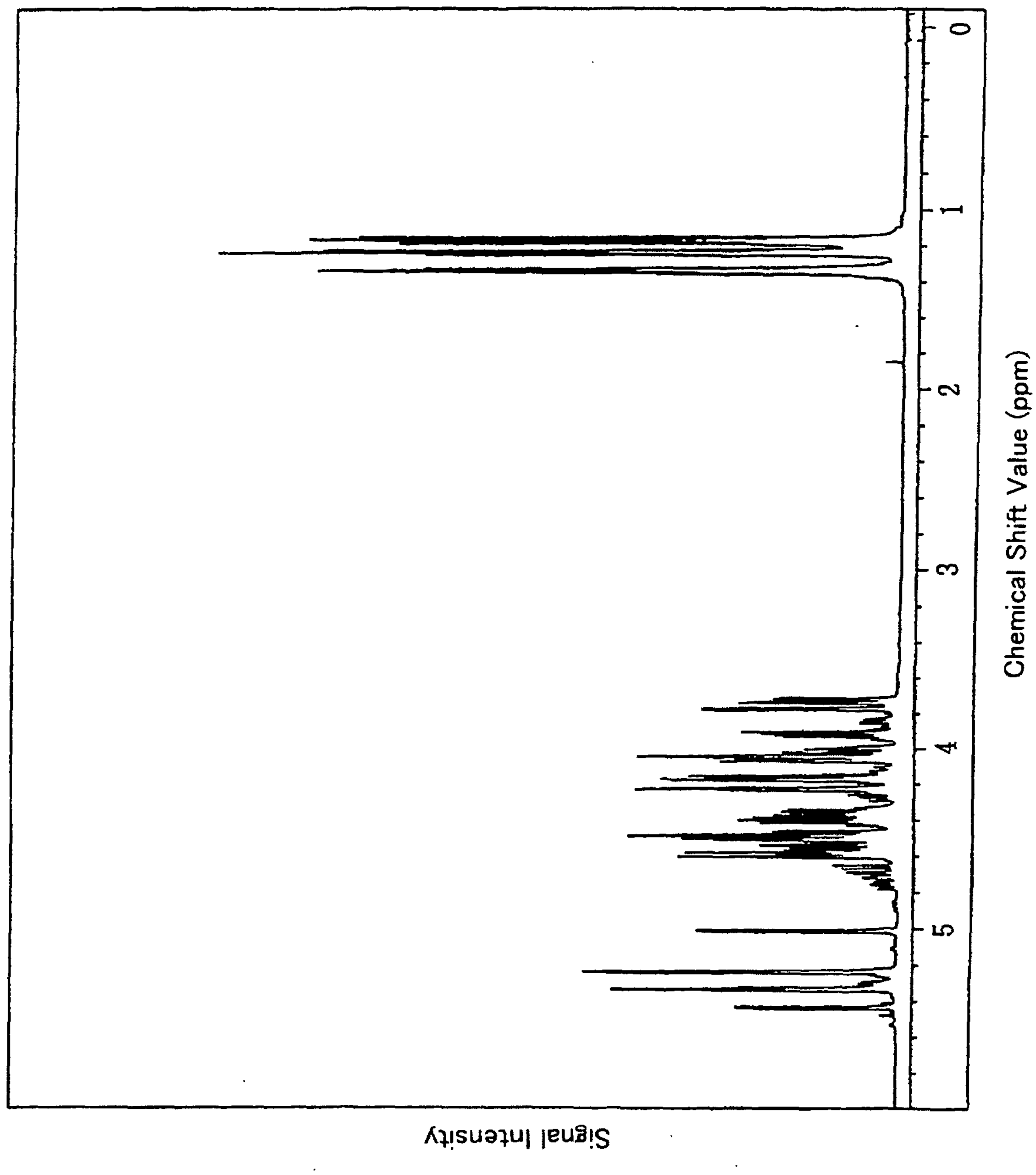

Fig. 10

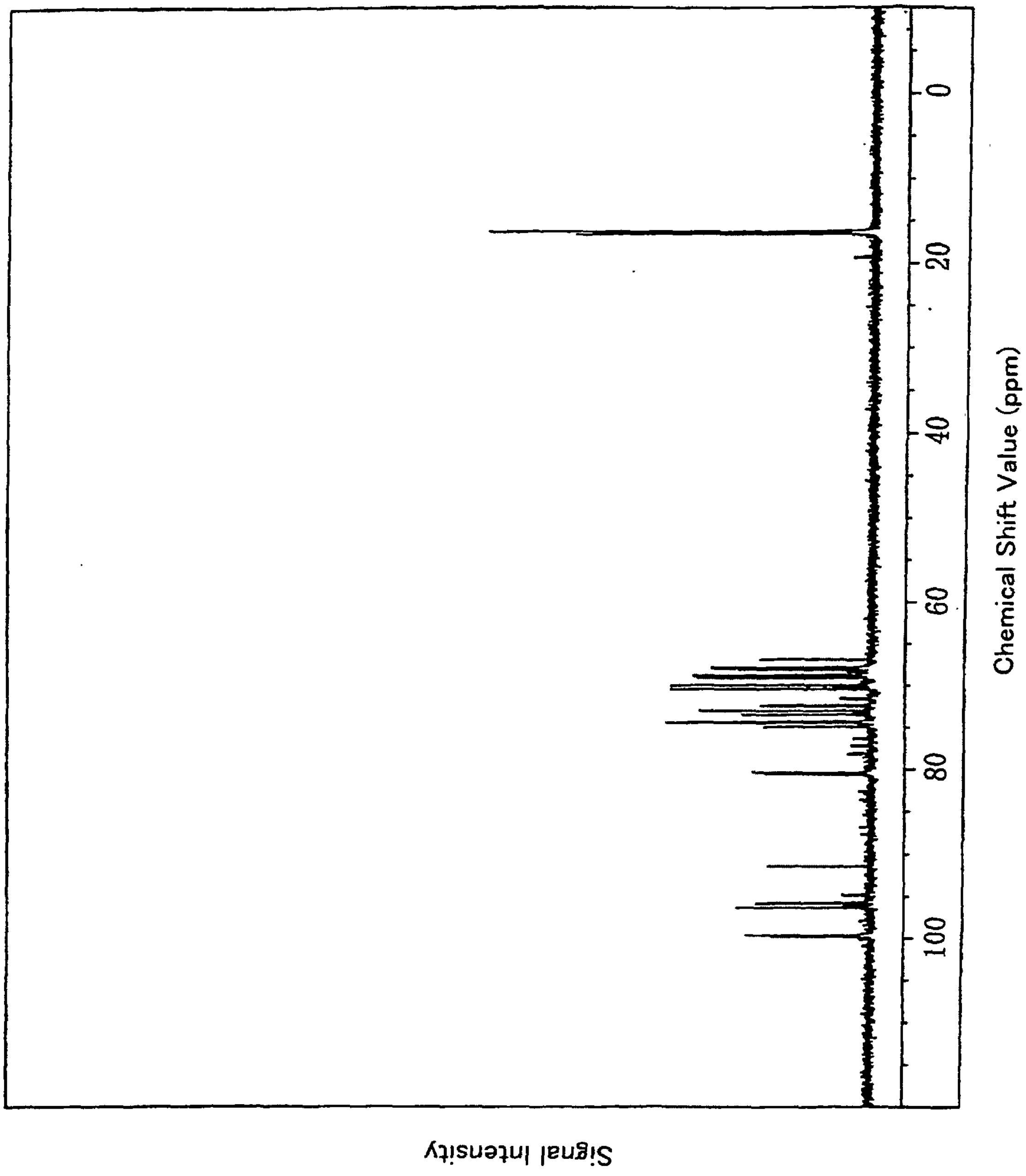

Fig. 11

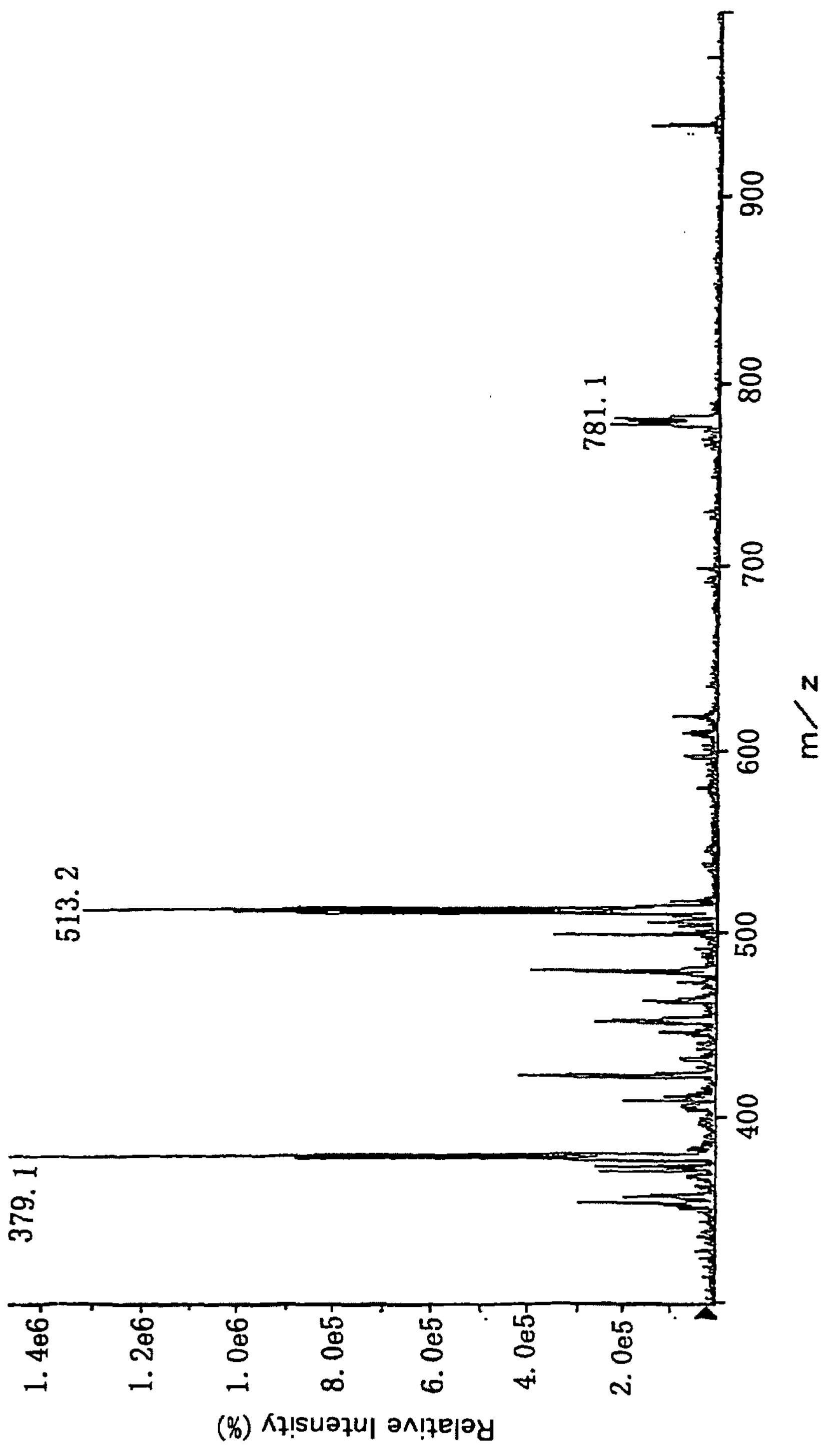
379.1
513.2
781.1
1.4e6
1.2e6
1.0e6
8.0e5
6.0e5
4.0e5
2.0e5
Relative Intensity (%)
400
500
600
700
800
900
m/z

Fig. 12

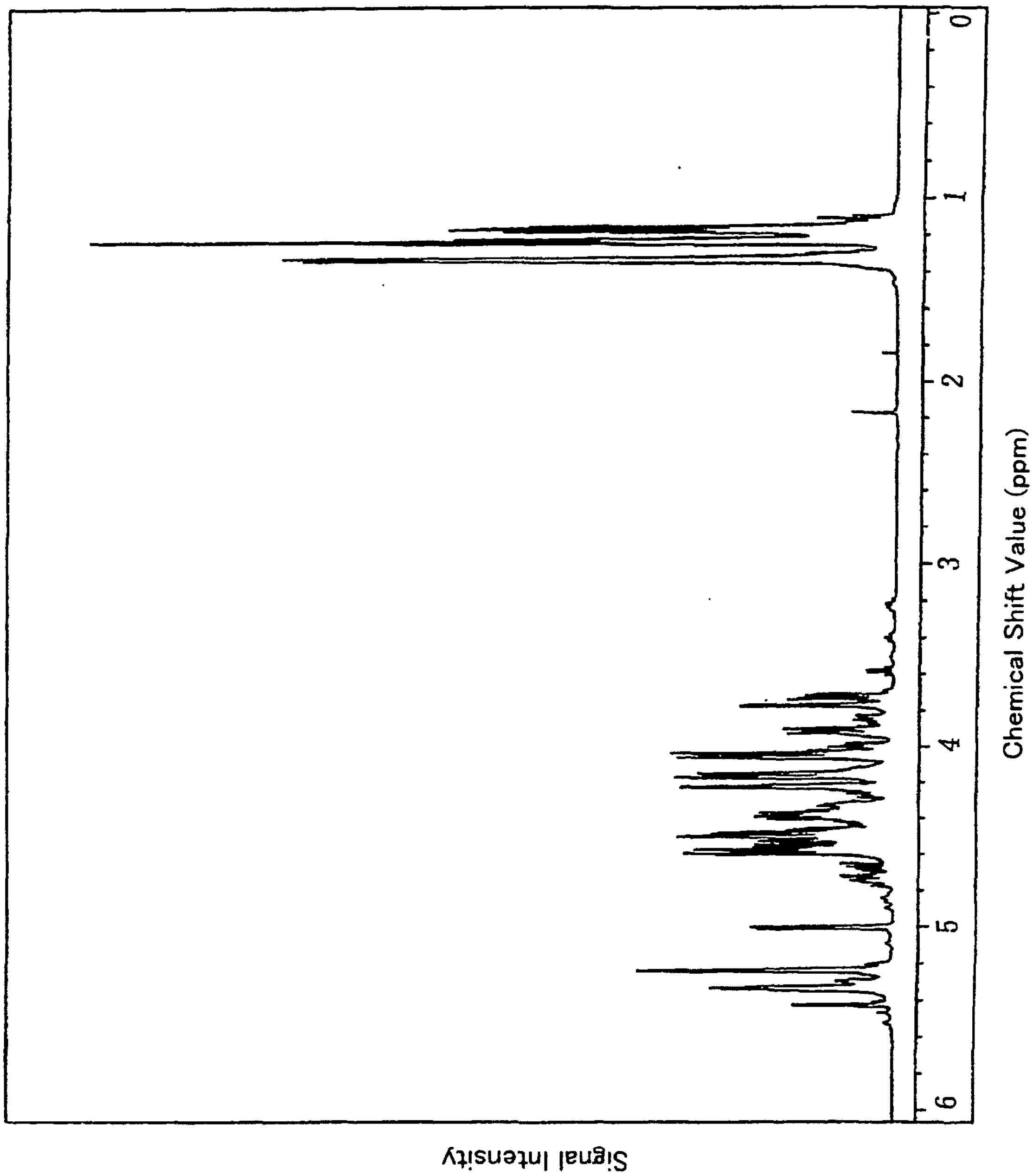

Fig. 13

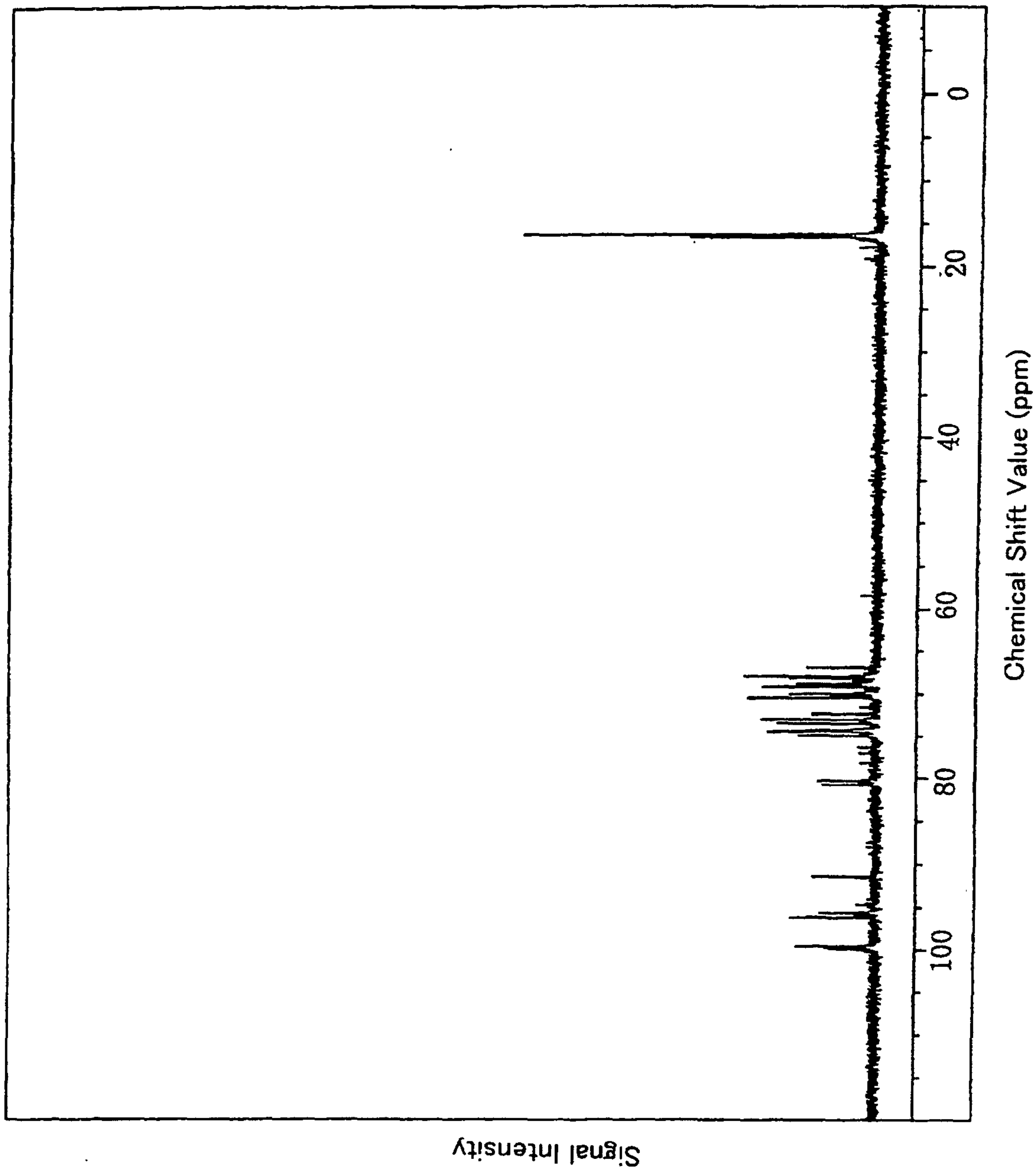

Fig. 14

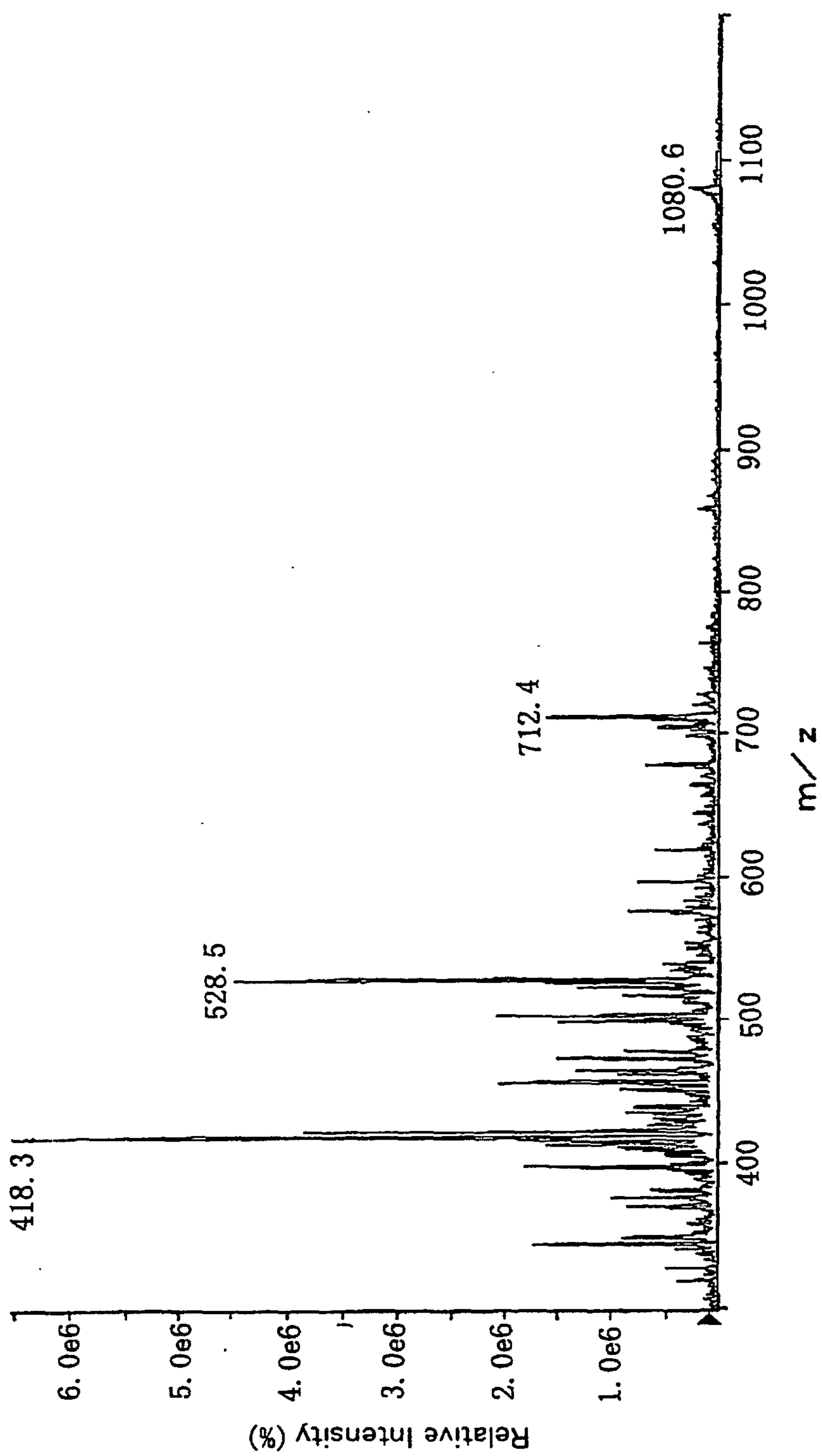

418.3
528.5
712.4
1080.6
400
500
600
700
800
900
1000
1100
m/z
6.0e6
5.0e6
4.0e6
3.0e6
2.0e6
1.0e6
Relative Intensity (%)

Fig. 15

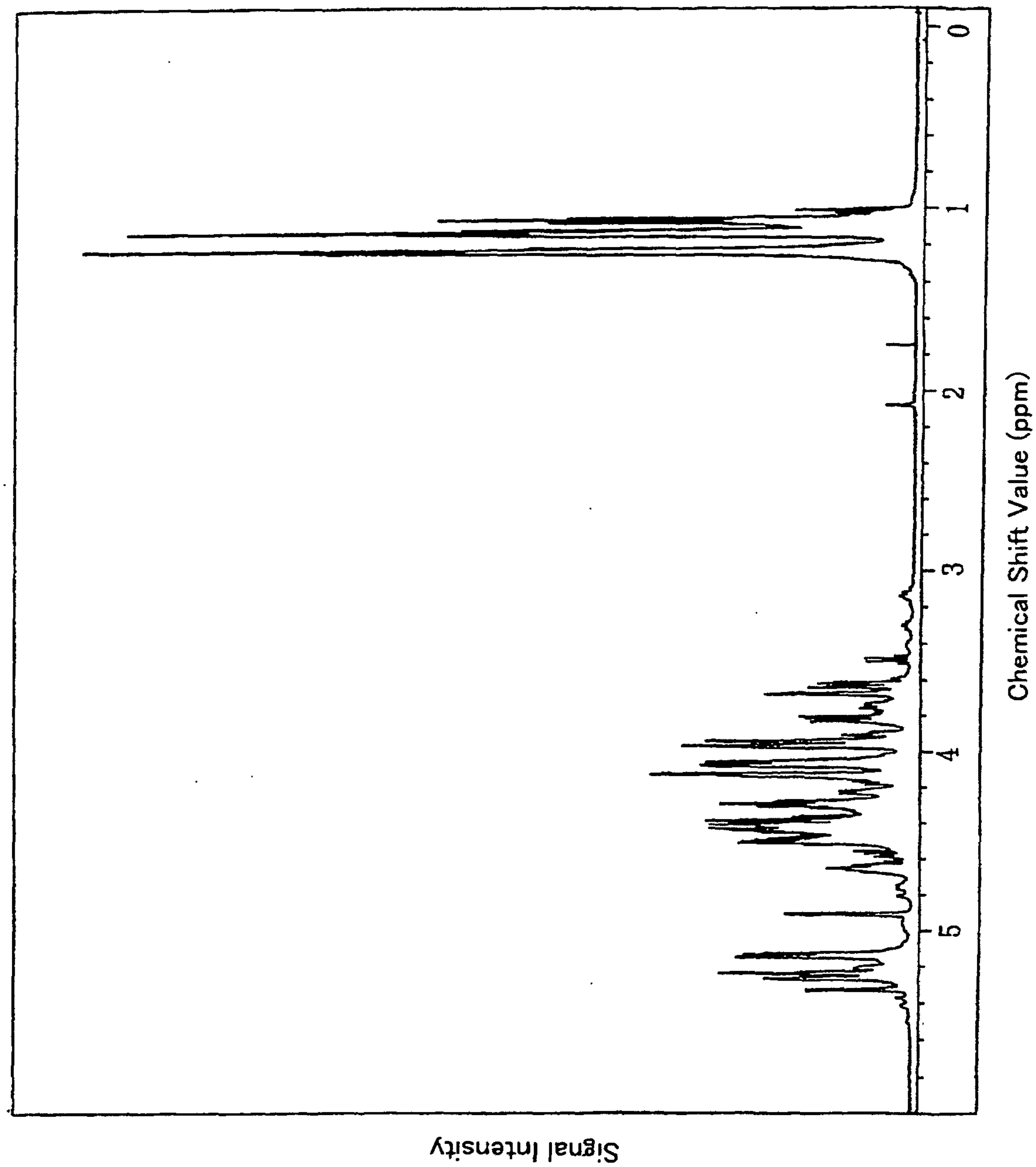

Fig. 16

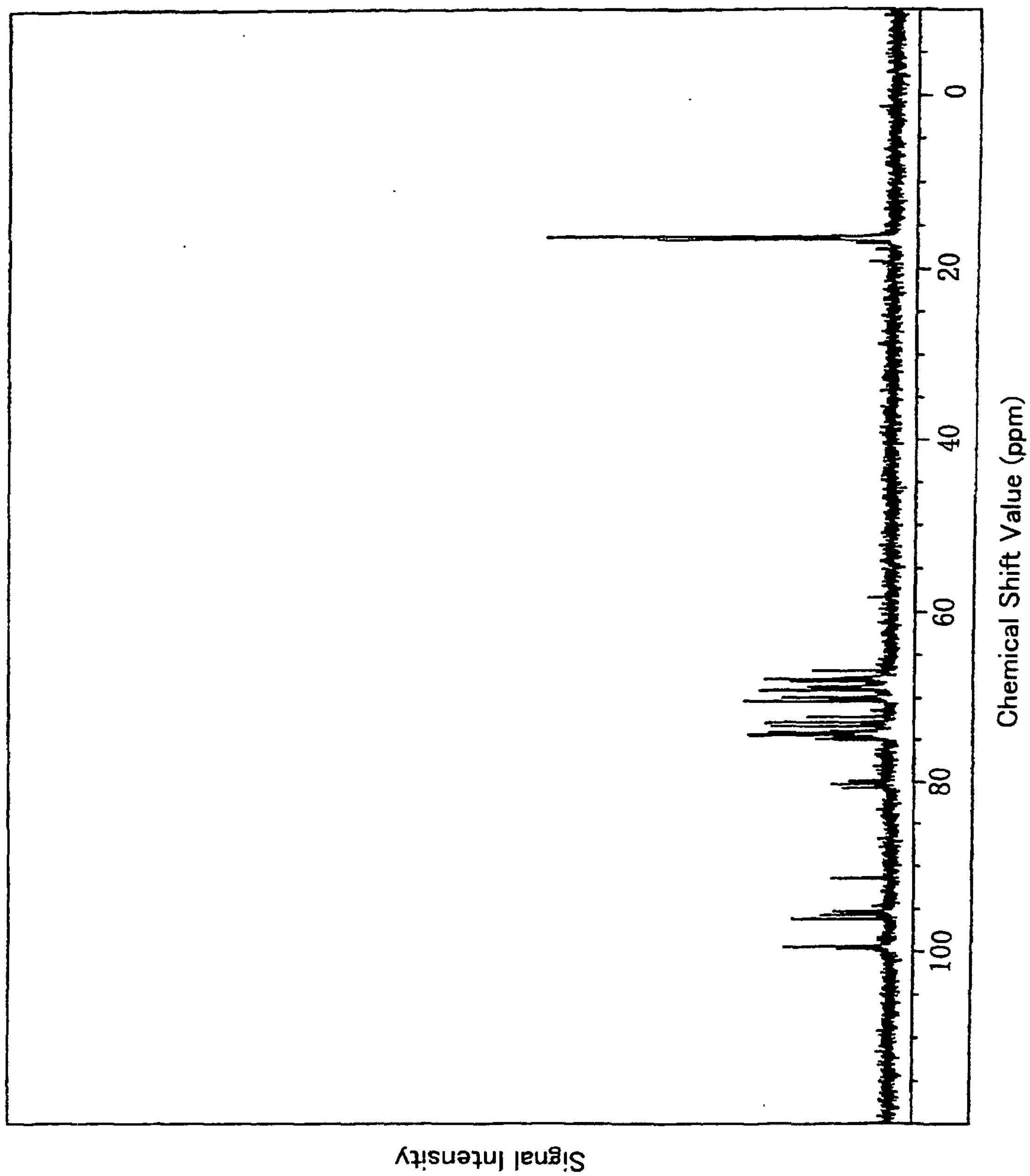

Fig.17

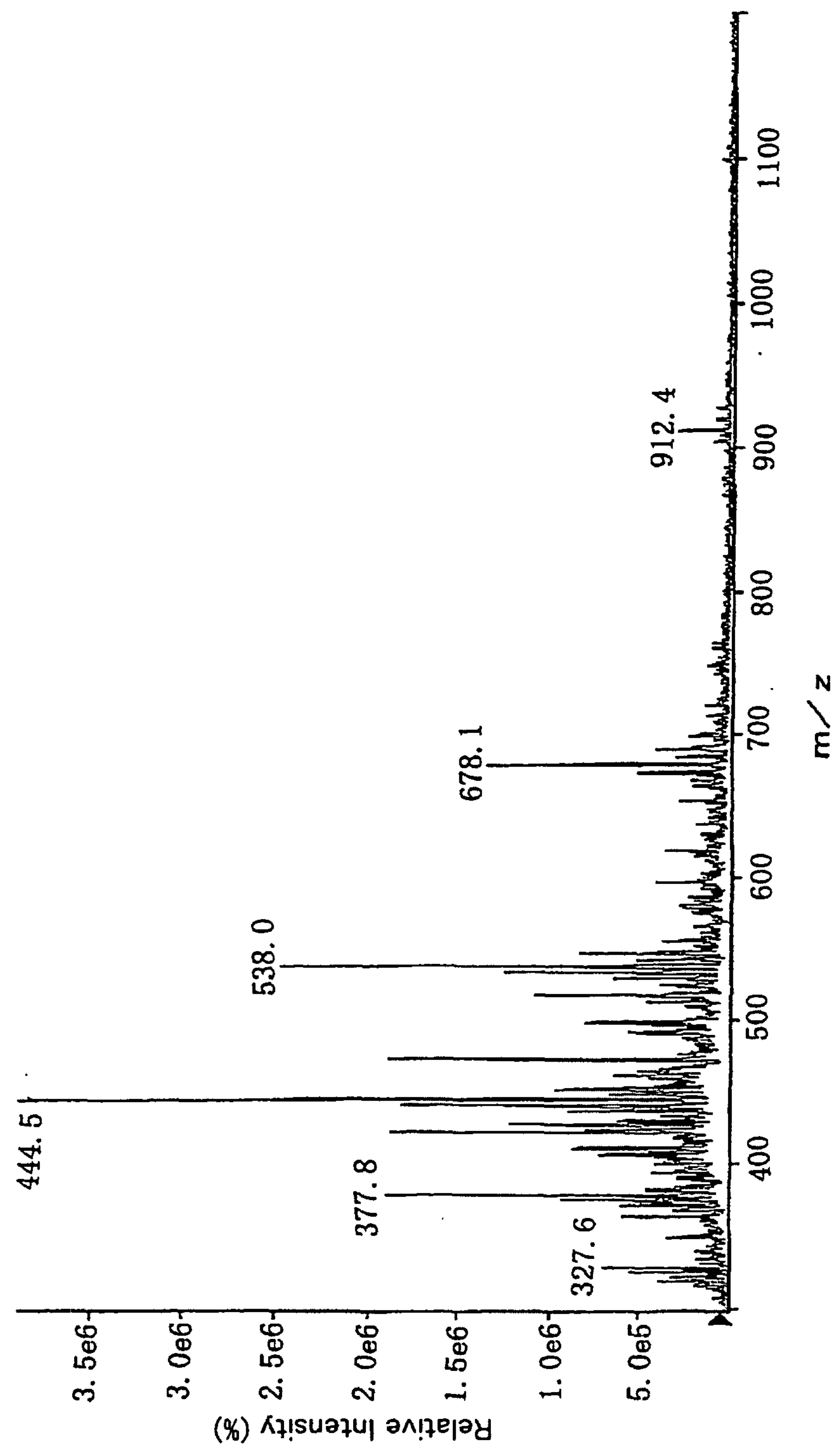
444.5
377.8
327.6
538.0
678.1
912.4
3.5e6
3.0e6
2.5e6
2.0e6
1.5e6
1.0e6
5.0e5
Relative Intensity (%)
400
500
600
700
800
900
1000
1100
m/z

Fig. 18

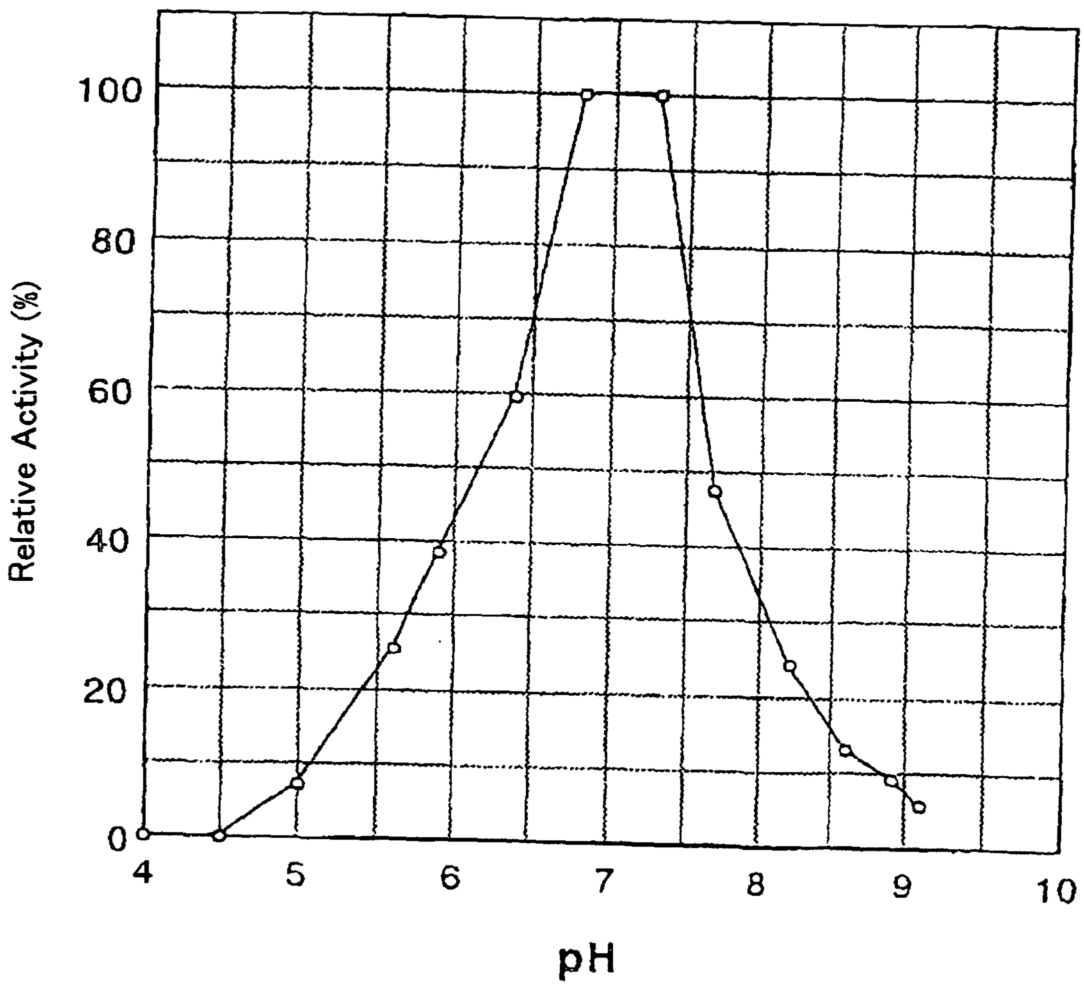

Fig. 19

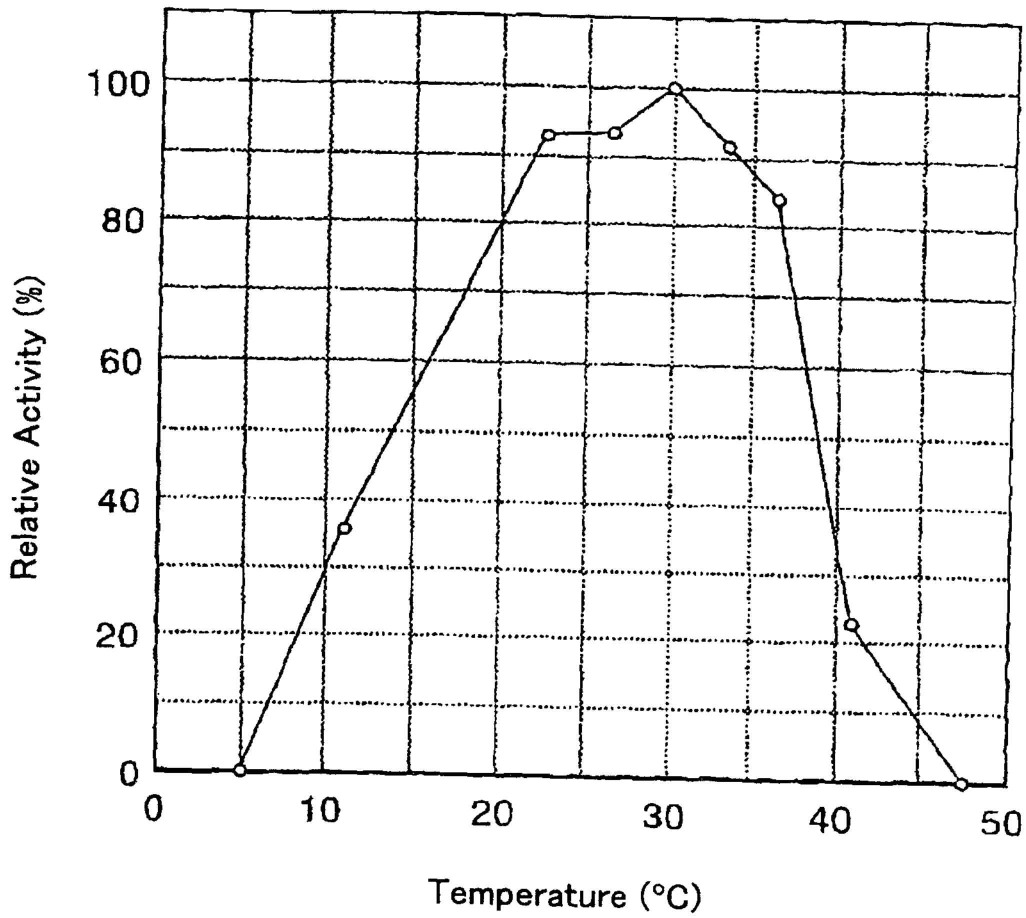
100
80
60
40
20
0
Relative Activity (%)
0
10
20
30
40
50
Temperature (°C)

## INTERNATIONAL SEARCH REPORT

International application No. PCT/JP03/00334

**A. CLASSIFICATION OF SUBJECT MATTER**
Int.Cl$^7$ C12N9/42, C07H11/00, C08B37/00, C12P19/04

According to International Patent Classification (IPC) or to both national classification and IPC

**B. FIELDS SEARCHED**

Minimum documentation searched (classification system followed by classification symbols)
Int.Cl$^7$ C12N9/42, C07H11/00, C08B37/00, C12P19/04

Documentation searched other than minimum documentation to the extent that such documents are included in the fields searched

Electronic data base consulted during the international search (name of data base and, where practicable, search terms used)
JSTPlus(JOIS), WPI(DIALOG),BIOSIS(DIALOG)

**C. DOCUMENTS CONSIDERED TO BE RELEVANT**

| Category* | Citation of document, with indication, where appropriate, of the relevant passages | Relevant to claim No. |
|---|---|---|
| X | WO 01/81560 A1 (Takara Shuzo Kabushiki Kaisha), 11 January, 2001 (11.01.01), Full text & EP 1277834 A1 | 1-9 |
| X | Sakai T. et al., Novel enzyme, fucoidan deacetylase, and the pathway of enzymatic degradation of fucoidan from Cladosiphon okamuranus, Abs. XXIIst Jap. Carbohydr. Symp., 2001, page 42 | 1-9 |
| X | Sakai T. et al., Two novel enzymes from a sea bacterium, α-D-glucuronidase and endo-α-L-fucosidase and their use for the analysis of the structure of fucoidan (sulfated glucuronofucan) from Cladosiphon okamuranus, Abs. XXIst Jap. Carbohydr. Symp. 2000, page 64 | 1-9 |

[X] Further documents are listed in the continuation of Box C. [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 10 April, 2003 (10.04.03) | 22 April, 2003 (22.04.03) |
| Name and mailing address of the ISA/ Japanese Patent Office | Authorized officer |
| Facsimile No. | Telephone No. |

Form PCT/ISA/210 (second sheet) (July 1998)

**INTERNATIONAL SEARCH REPORT**

International application No.
PCT/JP03/00334

C (Continuation). DOCUMENTS CONSIDERED TO BE RELEVANT

| Category* | Citation of document, with indication, where appropriate, of the relevant passages | Relevant to claim No. |
|---|---|---|
| X | WO 90/15823 A1 (Ifremer-Institut Francais De Recherche Pour L'exploitation De La Mer), 27 December, 1990 (27.12.90), Full text & EP 403377 B1 & US 5321133 A & JP 3042543 B2 | 4,6,7 |
| X | Chevolot L. et al., A disaccharide repeat unit is the major structure in fucoidans from two species of brown algae, Carbohydr. Res., 2001, Vol.330, No.4, pages 529-35 | 4,6,7 |
| P,X | Takeshi SAKAI et al., "Kaiso Yurai Fukoidan to sono Oligo-to no Kozo to Seibutsu Kassei", Bioscience & Industry, 01 June, 2002 (01.06.02), Vol.60, No.6, pages 377 to 380 | 1-9 |
| P,X | Hitomi KIMURA et al., "Shinki Fucoidanase o Riyo shite Kettei shita Fucus Vesiculosus Yurai Fucoidan no Shusa Kozo", Japanese Society for Marine Biotechnology Taikai Koen Yoshishu, 25 May, 2002 (25.05.02), Vol.6, page 105 | 1-9 |

Form PCT/ISA/210 (continuation of second sheet) (July 1998)

SEQUENCE LISTING

<110> TAKARA BIO INC.

<120> Sulfated fucan

<130> 663620

<150> JP 2002-10844

<151> 2002-01-18

<150> JP 2002-149874

<151> 2002-05-23

<160> 1

<210> 1

<211> 1517

<212> DNA

<213> Fucophilus fucoidanolyticus SI-1234

<400> 1

agagtttgat cctggctcag agtgaacgct ggcggcgtgg ttaagacatg caagtcgaac 60

gagattcttt gtattgaagc ctcggtggat ttataaagat gaaagtggca aacgggtgcg 120

taacacgtga gcaatctgcc ctaaagatcg gaatagctcg aggaaactcg aattaatgcc 180

ggatgtgata cgccaactca tgttggtagt attaaagctt gtaatggcgc tttaggagga 240

gctcgcggcc tatcagcttg ttggtgaggt aaaggctcac caaggcaaag acgggtagct 300

ggtctgagag gatgatcagc cacactggaa ctgagacacg gtccagacac ctacgggtgg 360

cagcagtttc gaatcattca caatgggggc aaccctgatg gtgcaacgcc gcgtgaggga 420

```
tgaaggcctt cgggtcgtaa acctctgtca ccagggagca acaagcaggt tcatagcctg   480
ccctgagtta acctggagag gaagcagtgg ctaactccgt gccagcagcc gcggtaatac   540
ggagactgca agcgttactc ggattcactg ggcgtaaagg gtgcgtaggc ggatagatgt   600
gtcaggtgtg aaatctcggg gctcaacctc gaaactgcgc ctgaaactgt ctatctagag   660
tattggaggg gtaagcggaa tttctggtgt agcggtgaaa tgcgtagata tcagaaggaa   720
caccaatggc gaaggcagct tactggacaa atactgacgc tgaggcacga aagcatgggt   780
agcgaaaggg attagatacc cctgtagtcc atgccgtaaa cgttgcacac taggtcttgg   840
gggtttcgac cctttcagga ccccagctaa cgcgataagt gtgccgcctg aggactacgg   900
ccgcaaggct aaaactcaaa ggaattgacg ggggcccgca caagcggtgg agcatgtggt   960
ttaattcgat gcaacgcgaa gaaccttacc taggcttgac atgtaatgga cgattttcag  1020
agatgaattt ttcccttcgg ggctgttaca caggtgctgc atggccgtcg tcagctcgtg  1080
tcgtgagatg tttggttaag tccagcaacg agcgcaaccc tcgtccttag ttgccagcac  1140
gtaatggtgg ggactctaag gagacaaact ctctttgaga gtgggaaggt ggggatgacg  1200
tcaggtcagt atggccctta cgcctagggc tacacacgtg ctacaatgcc cggtacaata  1260
ggacgcaata ccgcgaggtg gagcaaatcc tcaaaaccgg gcccagttcg gattggagtc  1320
tgcaactcga ctccatgaag tcggaatcgc tagtaatgac gtatcagcta tgacgtcgtg  1380
aatacgttcc cgggccttgt acacaccgcc cgtcacatca tgaaagccgg ttttgcccga  1440
agtacgtgag ctatccctcg ggaggcagcg tcctaaggca gggctggtga ttgggatgaa  1500
gtcgtaacaa ggtagcc   1517
```